# EUROPEAN PATENT APPLICATION

(11) **EP 2 471 794 A1**
(43) Date of publication of application: **04.07.2012**
(21) Application number: 10812099.9
(22) Date of filing: 26.08.2010
(51) Int. Cl.: C07D 493/04, C07D 317/70, G02B 5/23

(54) **CHROMENE COMPOUND**

(30) Priority: 28.08.2009 JP 2009198343
(71) Applicant: Tokuyama Corporation, Shunan-shi, Yamaguchi 745-0053 (JP)
(72) Inventor: TAKAHASHI Toshiaki, Shunan-shi Yamaguchi 745-0053 (JP); TAKENAKA Junji, Shunan-shi Yamaguchi 745-0053 (JP)
(74) Representative: Adam, Holger
(86) International application number: PCT/JP2010/064983
(87) International publication number: WO 2011/025056

(57) **Abstract**

A chromene compound having a skeleton represented by the following formula (1) and exhibiting double peak characteristic: 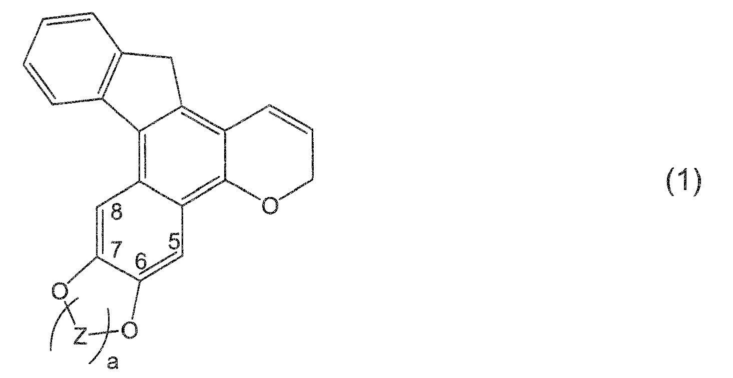 wherein Z is a group represented by any one of the following formulas: 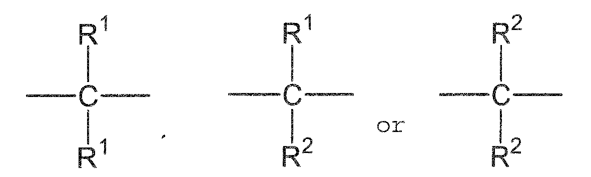 (R¹ is an electron donor group having a Hammett constant σₚ of less than -0.20, with the proviso that when there are a plurality of R¹'s, R¹'s may be the same or different, and R² is a group having a Hammett constant σₚ of -0.20 to 0, with the proviso that when there are a plurality of R²'s, R²'s may be the same or different)
and "a" is an integer of 1 to 3, with the proviso that when "a" is 2 or 3, Z's may be the same or different but 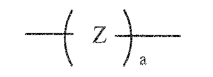 cannot be

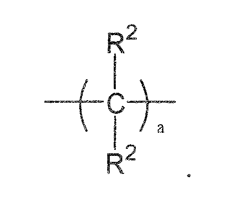

## Description

### TECHNICAL FIELD

The present invention relates to a novel chromene compound, and use and an intermediate thereof.

### BACKGROUND ART

Photochromism is the reversible function of a certain compound that it changes its color swiftly upon exposure to light including ultraviolet light such as sunlight or light from a mercury lamp and returns to its original color when it is put in the dark by stopping its exposure to light. A compound having this property is called "photochromic compound" and used as a material for photochromic plastic lenses and photochromic optical articles.

For the photochromic compound used for this purpose, the following properties are required: (i) the degree of coloration at a visible light range before ultraviolet light is applied (initial coloration) should be low, (ii) the degree of coloration upon exposure to ultraviolet light (to be referred to as "color optical density" hereinafter) should be high, (iii) the speed from the time when the application of ultraviolet light is started to the time when the color optical density reaches saturation (to be referred to as "color development sensitivity" hereinafter) should be high; (iv) the speed from the stoppage of the application of ultraviolet light to the time when the compound returns to its original state (to be referred to as "fading speed" hereinafter) should be high, (v) the repeat durability of this reversible function should be high, and (vi) the solubility in a monomer composition which will become a host material after curing of the photochromic compound should be high so that its dispersibility in the host material in use becomes high.

Since it is desired that photochromic plastic lenses should develop a color of a neutral tint such as gray or brown, as a matter of course, what color is developed is a very important factor for photochromic compounds. When the color is to be adjusted by mixing together a plurality of photochromic compounds, there occur various problems such as a color change at the time of fading (to be referred to as "color shift" hereinafter) due to the different characteristic properties of the photochromic compounds that are mixed together and a color change at the time of deterioration due to the difference in durability. To solve the above problems, a photochromic compound which by itself has two color development peaks when developing a color and develops a color of a neutral tint (to be referred to as "double peak compound" hereinafter) is important.

As the double peak compound, there are known a chromene compound represented by the following formula (A) (refer to a pamphlet of International Laid-Open WO2001/19813), a chromene compound represented by the following formula (B) or (C) (refer to a pamphlet of International Laid-Open WO2003/025638 , a chromene compound represented by the following formula (D) (refer to a pamphlet of International Laid-Open WO2003/044022) and a chromene compound represented by the following formula (E) (refer to a pamphlet of International Laid-Open WO2005/028465).

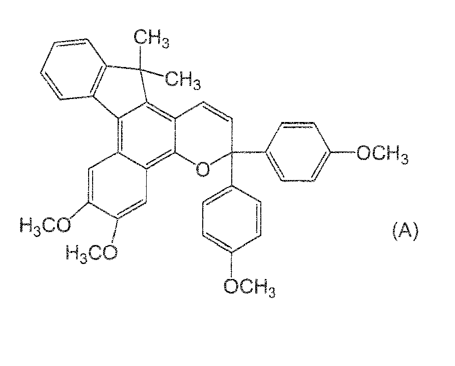

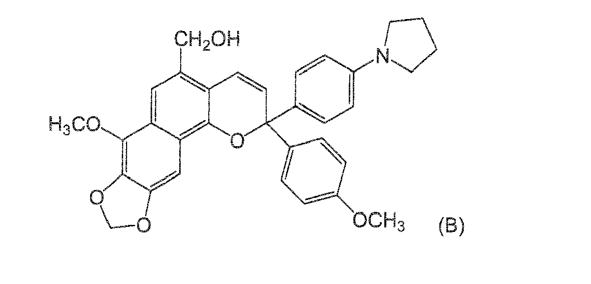

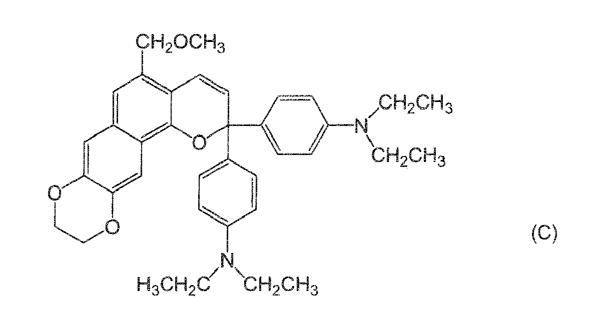

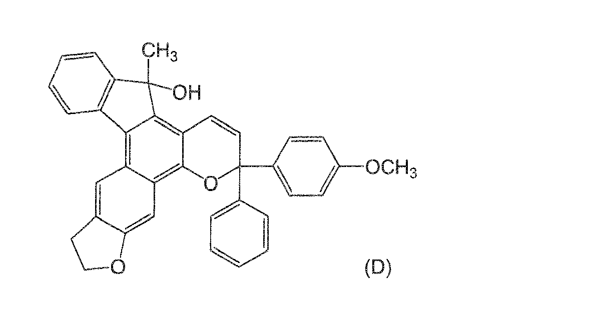

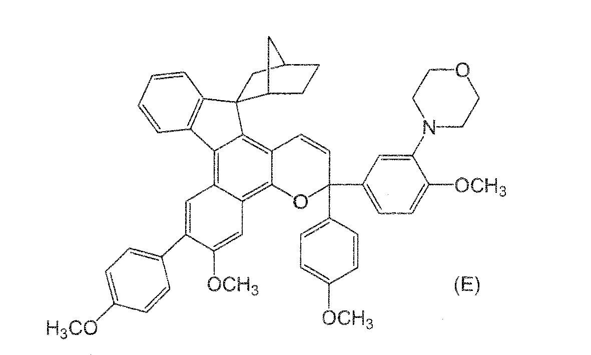

### DISCLOSURE OF THE INVENTION

In the field of photochromic plastic lenses, the requirements for photochromic properties, especially high fading speed when a photochromic plastic lens moves from outdoors to indoors and transparency when a user wears a photochromic plastic lens indoors (little initial coloration) are becoming stronger and stronger each year. Therefore, the development of a photochromic compound which satisfies all the above requirements (i) to (vi) at a higher level than the chromene compounds of the prior art is desired. When the color is to be adjusted by mixing together a plurality of photochromic compounds, it is known that a photochromic compound which develops a yellow color is generally inferior in durability to a photochromic compound which develops another color, for example, a blue color. Therefore, a compound having a higher yellow color optical density (having a maximum absorption wavelength at 430 to 530 nm) than the blue color optical density (having a maximum absorption wavelength at 550 to 650 nm) is desired as the double peak compound (the ratio of the yellow color optical density to the blue color optical density in the double peak compound may be referred to as "double peak characteristic" hereinafter). In consideration of these characteristic properties, the chromene compounds of the prior art have room for the improvement of the following points.

For example, although the chromene compound represented by the above formula (A) has practical levels of color optical density and double peak characteristic, it has room for improvement as it has a low fading speed. The chromene compounds represented by the above formula (B) , (C) and (D) also have room for improvement as they do not have satisfactory double peak characteristic. Further, although the chromene compound represented by the above formula (E) is excellent in double peak characteristic and has practical levels of color optical density and fading speed as it is a compound whose 7-position carbon atom is substituted by a specific aryl group, it has room for improvement as the end portion of its absorption spectrum (to be referred to as "absorption end" hereinafter) goes beyond 420 nm into the visible range with the result of large initial coloration.

Therefore, it is an object of the present invention to provide a novel photochromic compound (chromene compound) which has little initial coloration, high color optical density when it is exposed to light, high color development sensitivity, high fading speed and high durability and exhibits excellent double peak characteristic.

It is another object of the present invention to provide a naphthol compound which is an intermediate for the production of the chromene compound of the present invention.

Other objects and advantages of the present invention will become apparent from the following description.

The inventors of the present invention conducted intensive studies to attain the above objects and found the following fact. Although the double peak characteristic of a chromene compound having an indenonaphthopyran skeleton can be enhanced by increasing the electron donating abilities of the 6-position and 7-position substituents, when the electron donating abilities of the 6-position and 7-position substituents are increased, the fading speed becomes lower, the initial coloration becomes larger and the durability becomes lower in proportion to this.

Then, the inventors thought if the above disadvantage could be improved by adjusting the electron donating abilities of the 6-position and 7-position substituents while retaining the above advantage and investigated the introduction of various substituents.

They made further intensive studies and found that the above objects can be attained by a chromene compound having an indenonaphthopyran skeleton to which a hetero ring having oxygen atoms bonded to the 6-position and the 7-position is condensed and in which an electron donor group is introduced into the hetero ring. The present invention was accomplished based on this finding.

That is, firstly, the present invention is a chromene compound having a skeleton represented by the following formula (1): 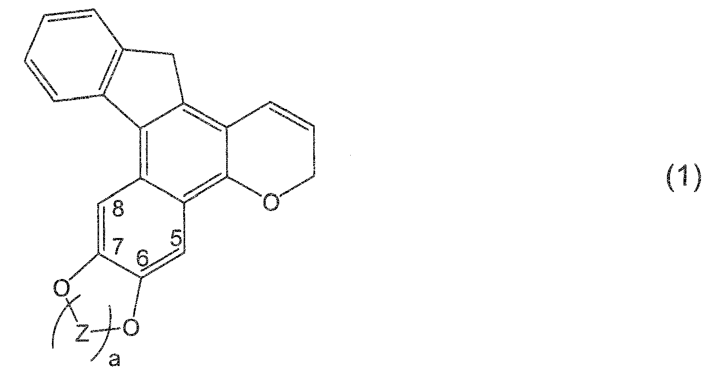 wherein Z is a group represented by any one of the following formulas: 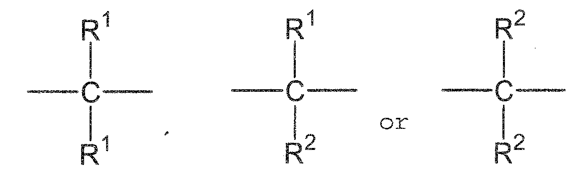 , wherein R¹ is an electron donor group having a Hammett constant σₚ of less than -0.20, with the proviso that when there are a plurality of R¹' s, R¹' s may be the same or different, and R² is a group having a Hammett constant σₚ of -0.20 to 0, with the proviso that when there are a plurality of R²'s, R²'s may be the same or different,
and "a" is an integer of 1 to 3, with the proviso that when "a" is 2 or 3, Z's may be the same or different but 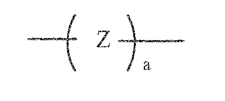 cannot be

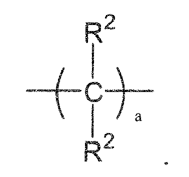

Secondly, the present invention is a photochromic curable composition which comprises the above chromene compound and a polymerizable monomer.

Thirdly, the present invention is a photochromic optical article which has a polymer molded product containing the above chromene compound dispersed therein as a constituent member of the photochromic optical article.

In the fourth place, the present invention is an optical article comprising an optical substrate which is at least partially coated with a polymer film as a constituent part of the optical particle, wherein the polymer film contains the above chromene compound dispersed therein.

In the fifth place, the present invention is a naphthol compound which is an intermediate for the production of the above chromene compound of the present invention and represented by the following formula (3): 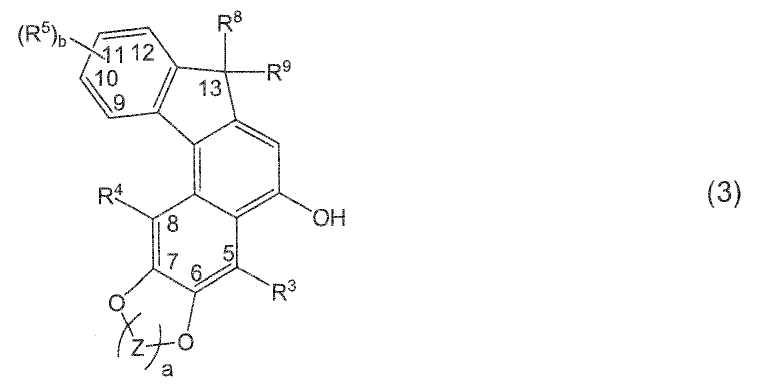 wherein Z and a are as defined in the above formula (1) , and R³, R⁴, R⁵, R⁸, R⁹ and b are as defined in the formula (2) in claim 2.

### BEST MODE FOR CARRYING OUT THE INVENTION

The chromene compound of the present invention is represented by the above formula (1).

As understood from the definition of Z in the formula (1) , in the formula (1), 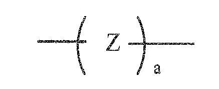 must be a group having at least one electron donor group (R¹) with a σₚ of less than -0.20.

A description is subsequently given of the group R¹ in the above group Z.

### (group R¹)

In the group Z, the group R¹ is an electron donor group having a σₚ of less than -0.20.

σₚ is defined based on the Hammett equation that quantifies the electric effect of a substituent bonded to an π electron system on the basis of the dissociation constant Ka of p-substituted benzoic acid. A substituent having a σₚ of 0 is a hydrogen atom, and a substituent having a σₚ of less than -0.20 is a substituent having high electron donating ability. When the group Z has this group R¹, the chromene compound of the present invention exhibits an excellent effect.

Examples of the electron donor group R¹ having a σₚ of less than -0.20 include hydroxyl group (σₚ = -0.37), alkoxy group, aralkoxy group, aryloxy group, amino group and heterocyclic group having a nitrogen atom as a ring member hetero atom and bonded to a carbon atom bonded thereto via the nitrogen atom. These groups may have an electron donor group having a σₚ of less than 0 as a substituent. Describing in detail hereinafter, the aralkoxy group, aryloxy group, amino group or heterocyclic group having a nitrogen atom as a ring member hetero atom and bonded to a carbon atom bonded thereto via the nitrogen atom is an electron donor group having a σₚ of less than -0.20 when it has no substituent. Therefore, these groups having an electron donor group having a σₚ of less than 0 as a substituent have a σ_{ρ} of less than -0.20 as well.

A detailed description is subsequently given of the above electron donor group having a σₚ of less than -0.20.

The alkoxy group generally has a σₚ of -0.30 or more to less than -0.20 and is preferably an alkoxy group having 1 to 8 carbon atoms in the present invention. Preferred examples of the alkoxy group include methoxy group (σₚ = -0.28) , ethoxy group (σₚ= -0.21), n-propoxygroup (σₚ= -0.26), isopropoxy group, n-butoxy group, sec-butoxy group and tert-butoxy group.

The aralkoxy group generally has a σₚ of less than -0.20 and is preferably an aralkoxy group having 7 to 11 carbon atoms in the present invention. Preferred examples of the aralkoxy group include benzyloxy group and naphthylmethoxy group. The aralkoxy group may be a group obtained by substituting one or more hydrogen atoms of a benzene ring by a group having a σₚ of less than 0, specifically the above or the following alkyl group, cycloalkyl group, alkoxy group, aralkyl group, aralkoxy group or aryloxy group. The aralkoxy group having any one of these substituents has a σₚ of less than -0.20 as well.

The aryloxy group generally has a σₚ of -0.6 to -.0.4 and is preferably an aryloxy group having 6 to 10 carbon atoms in the present invention. Preferred examples of the aryloxy group include phenyloxy group (σₚ = -0.49) and naphthyloxy group. The aryloxy group may be obtained by substituting one or more hydrogen atoms of a benzene ring by a group having a σₚ of less than 0, specifically the above and following alkyl group, cycloalkyl group, alkoxy group, aralkyl group, aralkoxy group or aryloxy group. The aryloxy group having any one of these substituents has a σₚ of less than -0.20 as well.

The amino group generally has a σₚ of -1.00 to -0.50 and is preferably a primary amino group (σₚ *=* -0.66) or a secondary amino group or tertiary amino group having a substituent. The substituent of the amino group is preferably a group having a σₚ of less than 0, typically an alkyl group. The amino group having the alkyl group as a substituent has a σₚ of less than -0.20 as well. Preferred examples of the substituted amino group, that is, the secondary amino group or the tertiary amino group include alkylamino groups such as methylamino group (σₚ = - 0.77) and ethylamino group; and dialkylamino groups such as dimethylamino group (σₚ = -0.83) and diethylamino group.

The heterocyclic group having a nitrogen atom as a ring member hetero atom and bonded to a carbon atom bonded thereto via the nitrogen atom generally has a σₚ of -1.00 to -0.40. Preferred examples of the heterocyclic group include morpholino group (σₚ = -0.50), piperidino group (σₚ = -0.83), pyrrolidinyl group, piperazino group, N-methylpiperadino group and indolinyl group. Further, the heterocyclic group may have a substituent having a σₚ of less than 0. Examples of the substituent include alkyl groups such as methyl group. Even when the heterocyclic group has such a substituent, it has a σₚ of less than -0.20. Examples of the heterocyclic group having a substituent include 2,6-dimethylmorpholino group, 2,6-dimethylpiperidino group and 2,2,6,6-tetramethylpiperidino group.

In the present invention, to enhance the double peak characteristic while retaining excellent fading sped, the group R¹ is an electron donor group having a σₚ of preferably -1.00 or more to less than -0.20, particularly preferably -0.80 or more to less than -0.20 out of the above examples. More specifically, R¹ is preferably a hydroxyl group, alkoxy group or heterocyclic group having a nitrogen atom as a ring member hetero atom and bonded to a carbon atom bonded thereto via the nitrogen atom, especially hydroxyl group, methoxy group or morpholino group.

### (group R²)

In the group Z, the group R² is an electron donor group having a σₚ of -0.20 to 0. Examples of R² having a σₚ of -0.20 to 0 include hydrogen atom, alkyl group, cycloalkyl group, aralkyl group and aryl group.

The alkyl group is generally a group having a σₚ of - 0 .20 to -0.10 and particular preferably an alkyl group having 1 to 8 carbon atoms in the present invention. Preferred examples of the alkyl group include methyl group (σₚ = -0 .14) , ethyl group (σₚ = -0.13), n-propyl group (σₚ = -0.12), isopropyl group, n-butyl group, sec-butyl group, tert-butyl group σₚ = -0.15), pentyl group, hexyl group, heptyl group and octyl group.

The cycloalkyl group is generally a group having a σₚ of -0.20 or more to less than 0 and particularly preferably a cycloalkyl group having 3 to 8 carbon atoms in the present invention. Preferred examples of the cycloalkyl group include cyclopropyl group, cyclobutyl group, cyclopentyl group, cyclohexyl group (σₚ = -0.16), cycloheptyl group and cyclooctyl group.

The aralkyl group is generally a group having a σₚ of -0.20 or more to less than 0 and particularly preferably an aralkyl group having 7 to 11 carbon atoms in the present invention. Preferred examples of the aralkyl group include benzyl group, phenyethyl group, phenylpropyl group, phenylbutyl group and naphthylmethyl group.

The aryl group is generally a group having a σₚ of -0 .10 to -0.01 and particularly preferably an aryl group having 6 to 14 carbon atoms in the present invention. Preferred examples of the aryl group include phenyl group (σₚ = -0.01) and 1-naphthyl group (σₚ = -0.08).

Out of these, the group R² is particularly preferably an alkyl group such as methyl group or a hydrogen atom because a compound having high durability is obtained and easily synthesized.

### (number "a" of group Z's)

"a" indicates the number of the group z's and an integer of 1 to 3. When "a" is 2 or 3, "a"'s may be the same or different.

### (preferred group Z)

In the present invention, in order to obtain a chromene compound which is excellent in fading speed and durability while retaining high double peak characteristic, the group Z is particularly preferably the following group.
When a=1, Z in which the total of the Hammett constants of the group R¹ and the group R¹ in 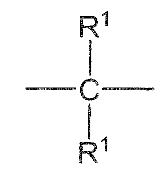 or the total of the Hammett constants of the group R¹ and the group R² in 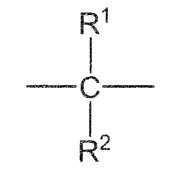 is from -1.00 or more to less than -0.20 is preferred, and Z in which the total of the above Hammett constants is from -0.80 or more to less than -0.20 is particularly preferred. When the total of the above Hammett constants is less than -1.00, the fading speed tends to become slow.
When a=2, Z in which the total of the Hammett constants of the group R¹ and the group R¹ in 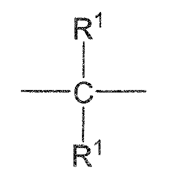 or the total of the Hammett constants of the group R¹ and the group R² in 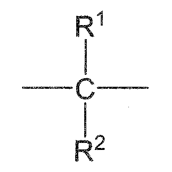 is from -1.00 or more to less than -0.20 is preferred, and Z in which the total of the above Hammett constants is from -0.80 or more to less than -0.20 is particularly preferred. When the total of the above Hammett constants is less than -1.00, the fading speed tends to become slow.

Out of the two Z' s, one in which the total of the Hammett constants of the group R¹ and the group R¹ or the group R¹ and the group R² is smaller may be bonded to either the oxygen atom bonded to the 6-position of the indenonaphthopyran skeleton or the oxygen atom bonded to the 7--position, preferably bonded to the oxygen atom bonded to the 7-position because higher double peak characteristic is obtained while the fading speed is maintained.

Further, when a=3, Z in which the total of the Hammett constants of the group R¹ and the group R² in 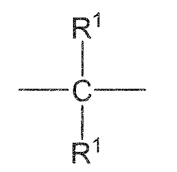 or the total of the Hammett constants of the group R¹ and the group R² in 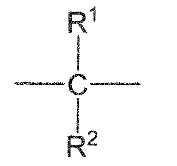 is from -1.00 or more to less than -0.20 is preferred, and Z in which the total of the above Hammett constants is from -8.0 or more to less than -0.20 is particularly preferred. When the total of the above Hammett constants is less than -1.00, the fading speed tends to become slow.

Out of the three Z's, one in which the total of the Hammett constants of the group R¹ and the group R¹ or the group R¹ and the group R² is the smallest is preferably bonded to either the oxygen atom bonded to the 6-position of the indenonaphthopyran skeleton or the oxygen atom bonded to the 7-position, particularly preferably bonded to the oxygen atom bonded to the 7-position because higher double peak characteristic is obtained while the fading speed is maintained.

"a" is preferably 1 or 2 from the viewpoints of double peak characteristic and fading speed. "a" is particularly preferably 1 from the viewpoint of fading speed and 2 from the viewpoint of double peak characteristic.

Particularly preferred combinations of Z and "a" are given below. In the following formulas, the carbon atoms at positions denoted by 6 and 7 are carbon atoms at the 6-position and the 7-position in the above formula (1).

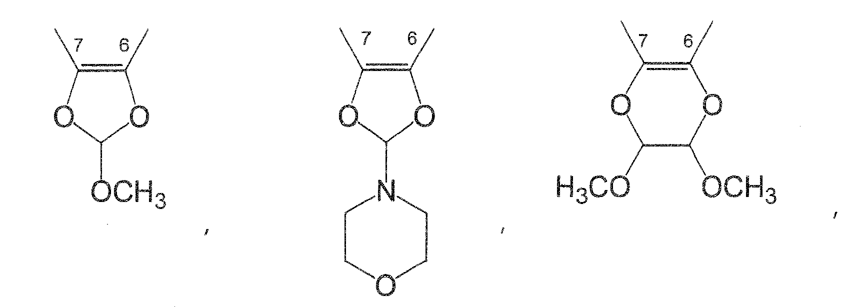

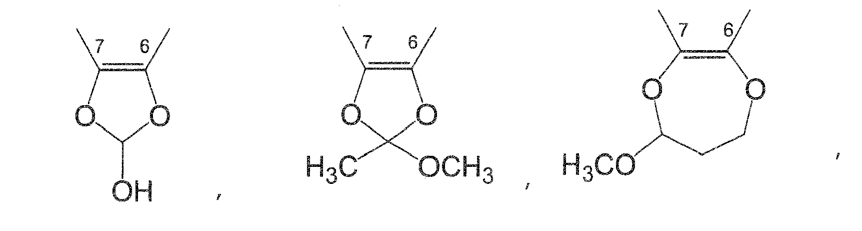

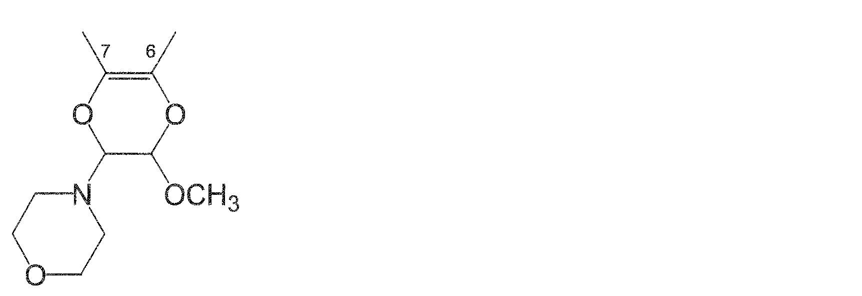

Since the Chromene compound of the present invention has an indenonaphthopyran skeleton as shown by the above formula (1) and has the above substituents at the 6-position and the 7-position, it exhibits excellent photochromic properties. Therefore, the other groups are not particularly limited. A preferred chromene compound which exhibits more excellent photochromic properties is the following chromene compound.

### (preferred chromene compound>

In the present invention, out of the Chromene compounds having a skeleton represented by the above formula (1), a chromene compound represented by the following formula. (2) is preferred.

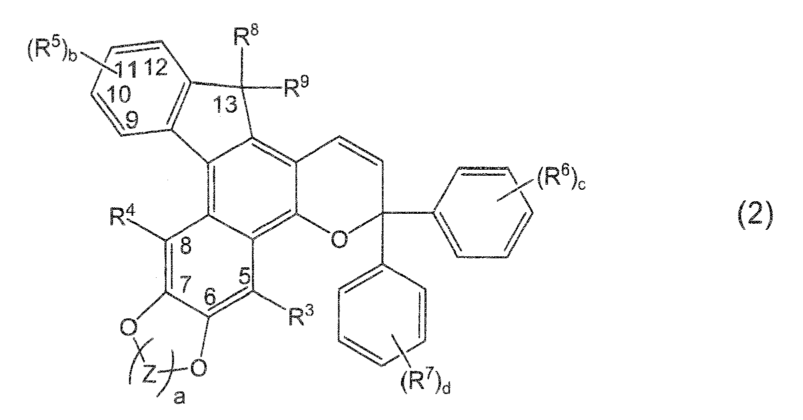

In the chromene compound represented by the above formula (2), Z and "a" are as defined in the above formula (1). A description is subsequently given of groups other than these groups.

### (groups R³ and R⁴)

In the chromene compound represented by the above formula (2) , R³ and R⁴ are each independently a hydrogen atom, hydroxyl group, alkyl group, haloalkyl group, alkenyl group, alkynyl group, cycloalkyl group, alkoxy group, aralkyl group, aralkoxy group, aryloxy group, aryl group, amino group, heterocyclic group having a nitrogen atom as a ring member hetero atom and bonded to the 5-position or 8-position carbon atom via the nitrogen atom, cyano group, nitro group, formyl group, hydroxycarbonyl group, alkylcarbonyl group, alkoxycarbonyl group or halogen atom.

Preferred examples of the above alkyl group, cycloalkyl group, alkoxy group, aralkyl group, aralkoxy group, aryloxy group, aryl group, amino group and heterocyclic group are the same as those enumerated for the above R¹ or R².

The haloalkyl group is preferably a haloalkyl group having 1 to 8 carbon atoms such as trifluoromethyl group or 2,2,2-trifluoroethyl group.

The alkenyl group is preferably an alkenyl group having 2 to 9 carbon atoms such as allyl group, propenyl group, 1-butenyl group or 2-butenyl group.

The alkynyl group is preferably an alkynyl group having 2 to 9 carbon atoms such as propargyl group or 1 -pentinyl group.

The alkylcarbonyl group is preferably an alkylcarbonyl group having 2 to 9 carbon atoms such as methylcarbonyl group or ethylcarbonyl group.

The alkoxycarbonyl group is preferably an alkoxycarbonyl group having 2 to 9 carbon atoms such as methoxycarbonyl group or ethoxycarbonyl group.

Preferred examples of the halogen atom are fluorine atom, chlorine atom, bromine atom and iodine atom.

In the present invention, the groups R³ and R⁴ are involved in fading speed. To accelerate the fading speed, R³ is preferably a stereoscopically small substituent, particularly preferably a hydrogen atom.

### (group R⁵)

In the chromene compound represented by the above formula (2), R⁵ is a hydroxyl group, alkyl group, haloalkyl group, alkenyl group, alkynyl group, cycloalkyl group, alkoxy group, aralkyl group, aralkoxy group, aryloxy group, aryl group, amino group, heterocyclic group having a nitrogen atom as a ring member hetero atom and bonded to a benzene ring bonded thereto via the nitrogen atom, cyano group, nitro group, formyl group, hydroxycarbonyl group, alkylcarbonyl group, alkoxycarbonyl group or halogen atom.

Examples of the above alkyl group, haloalkyl group, alkenyl group, alkynyl group, cycloalkyl group, alkoxy group, aralkyl group, aralkoxy group, aryloxy group, aryl group, amino group, heterocyclic group, alkylcarbonyl group, alkoxycarbonyl group and halogen atom are the same as those enumerated for the above R¹, R², R³ or R⁴.

"b" indicates the number of R⁵' s and is an integer of 0 to 4. When "b" is 2 to 4, R⁵' s may be the same or different.

In the present invention, the group R⁵ is involved in fading speed. The group R⁵ is preferably a hydrogen atom ("b" is 0) or an electron absorbing group. When the group R⁵ is an electron absorbing group, to accelerate the fading speed, the group R⁵ is preferably bonded to the 11-position carbon atom. The particularly preferred electron absorbing group is a cyano group or haloalkyl group, more specifically, cyano group or trifluoromethyl group.

### (groups R⁶ and R⁷)

In the chromene compound represented by the above formula (2) , R⁶ and R⁷ are each independently a hydroxyl group, alkyl group, haloalkyl group, alkenyl group, alkynyl group, cycloalkyl group, alkoxy group, aralkyl group, aralkoxy group, aryloxy group, aryl group, amino group, heterocyclic group having a nitrogen atom as a ring member hetero atom and bonded to a benzene ring bonded thereto via the nitrogen atom, cyano group, nitro group, formyl group, hydroxycarbonyl group, alkylcarbonyl group, alkoxycarbonyl group or halogen atom. Preferred examples of the above alkyl group, haloalkyl group, alkenyl group, alkenyl group, cycloalkyl group, alkoxy group, aralkyl group, aralkoxy group, aryloxy group, aryl group, amino group, heterocyclic group, alkylcarbonyl group, alkoxycarbonyl group and halogen atom are the same as those enumerated for the above R¹, R² R³ or R⁴.

"c" and "d" indicate the numbers of the substituents R⁶ and R⁷, respectively, and are each independently an integer of 0 to 5. Whey "c" and "d" are each an integer of 2 to 5, R⁶'s and R⁷'s may be the same or different.

In the present invention, the substituents R⁶ and R⁷ are involved in double peak characteristic and fading speed. Although the numbers and positions of the substituents are not particularly limited, they are preferably hydrogen atoms ("c" and/or "d" are/is 0) or existent at the p-position in order to obtain high double peak characteristic and a high fading speed. The preferred substituents are each a hydrogen atom ("c" and/or "d" are/is 0), alkyl group, alkoxy group, amino group or heterocyclic group having a nitrogen atom as a ring member hetero atom and bonded to a benzene ring bonded thereto via the nitrogen atom. Preferred examples of these substituents include methyl group, methoxy group, dimethylamino group and morpholino group.

### (groups R⁸ and R⁹)

In the chromene compound represented by the above formula (2), R⁸ and R⁹ are each independently a hydrogen atom, hydroxyl group, alkyl group, haloalkyl group, alkenyl group, alkynyl group, cycloalkyl group, alkoxy group, aralkyl group, aralkoxy group, aryloxy group, aryl group, amino group, heterocyclic group having a nitrogen atom as a ring member hetero atom and bonded to the 13-position carbon atom via the nitrogen atom, cyano group, nitro group, formyl group, hydroxycarbonyl group, alkylcarbonyl group, alkoxycarbonyl group or halogen atom.

Preferred examples of the above alkyl group, haloalkyl group, alkynyl group, alkynyl group, cycloalkyl group, alkoxy group, aralkyl group, aralkoxy group, aryloxy group, aryl group, amino group, heterocyclic group, alkylcarbonyl group, alkoxycarbonyl group and halogen atom are the same as those enumerated for the above R¹, R², R³ or R⁴.

In the chromene compound represented by the above formula (2) , R⁸ and R⁹ may be bonded together to form a carbonyl group or an aliphatic hydrocarbon ring together with the 13-position carbon atom.

The number of the ring member carbon atoms of the aliphatic hydrocarbon ring is preferably 4 to 20, more preferably 4 to 15 from the viewpoints of color optical density and fading speed. From the viewpoint of fading speed, the number of ring member carbon atoms is particular preferably 4 to 12. This aliphatic hydrocarbon ring may have at least one substituent selected from the group consisting of alkyl group, haloalkyl group, cycloalkyl group, alkoxy group, amino group, aralkyl group, aryl group and halogen atom.

Preferred examples of the alkyl group, haloalkyl group, cycloalkyl group, alkoxy group, amino group, aralkyl group, aryl group and halogen atom as substituents are the same as those enumerated for the above R¹, R², R³ or R⁴. Out of these, an alkyl group is preferred from the viewpoints of color optical density and fading speed, as exemplified by methyl group.

In the present invention, preferred substituents R⁸ and R⁹ are each selected from alkyl group, alkoxy group and hydroxyl group, or R⁸ and R⁹ are bonded together to form an aliphatic hydrocarbon ring together with the 13-position carbon atom. An example of the alkyl group is a methyl group, and an example of the alkoxy group is a methoxy group. Out of the above preferred substituents, R⁸ and R⁹ are preferably bonded together to form an aliphatic hydrocarbon ring together with the 13-position carbon atom so as to reduce color development by heat at room temperature under no exposure to light (this color development will be referred to as "initial coloration due to thermochromism" hereinafter) and accelerate the fading speed while retaining the double peak characteristic. The aliphatic hydrocarbon ring is preferably a single ring having 4 to 20 ring member carbon atoms, a bicyclo ring or a tricyclo ring. Specific examples of the aliphatic hydrocarbon ring include single rings such as cyclobutane ring, cyclopentane ring, cyclohexane ring, cycloheptane ring, cyclooctane ring, cyclononane ring, cyclodecane ring and 3,3,5,5-tetramethylcyclohexane ring, bicyclo rings such as bicycio[2,2,1]heptane ring, bicyclo[3,2,1]octane ring and bicyclo[3,3,1]nonane ring, and trioyclo rings such as adamantane ring.

Out of these, a single ring formed by bonding together the groups R⁸ and R⁹ exhibits a particularly excellent effect. Specific examples of the single ring include cyclobutane ring, cyclopentane ring, cyclohexane ring, cycloheptane ring, cyclooctane ring, cyclononane ring, cyclodecane ring and 3,3,5,5-tetramethylcyclohexane ring.

Out of the above single rings, cyclooctane ring and 3,3,5,5-tetramethylcyclohexane ring are particularly preferred.

### (preferred examples of chromene compound)

In the present invention, preferred examples of the chromene compound are the following compounds.

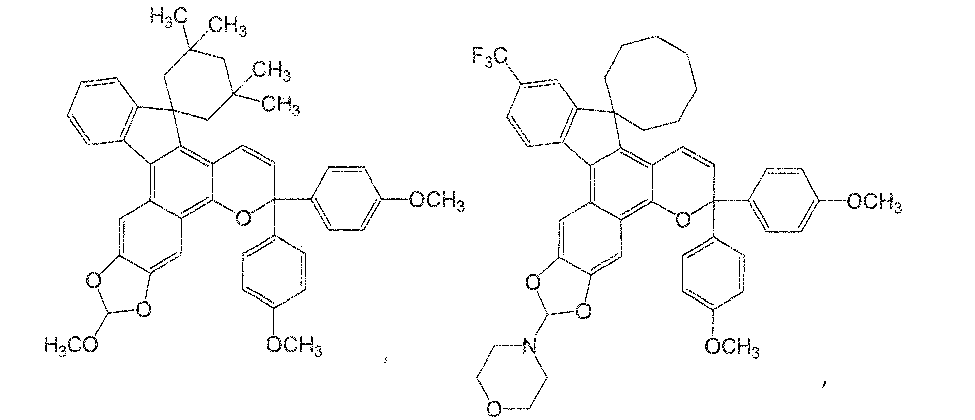

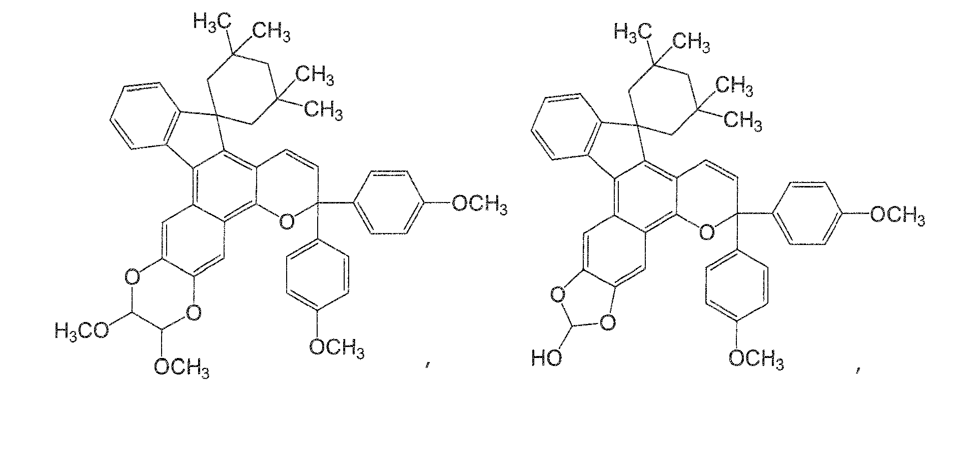

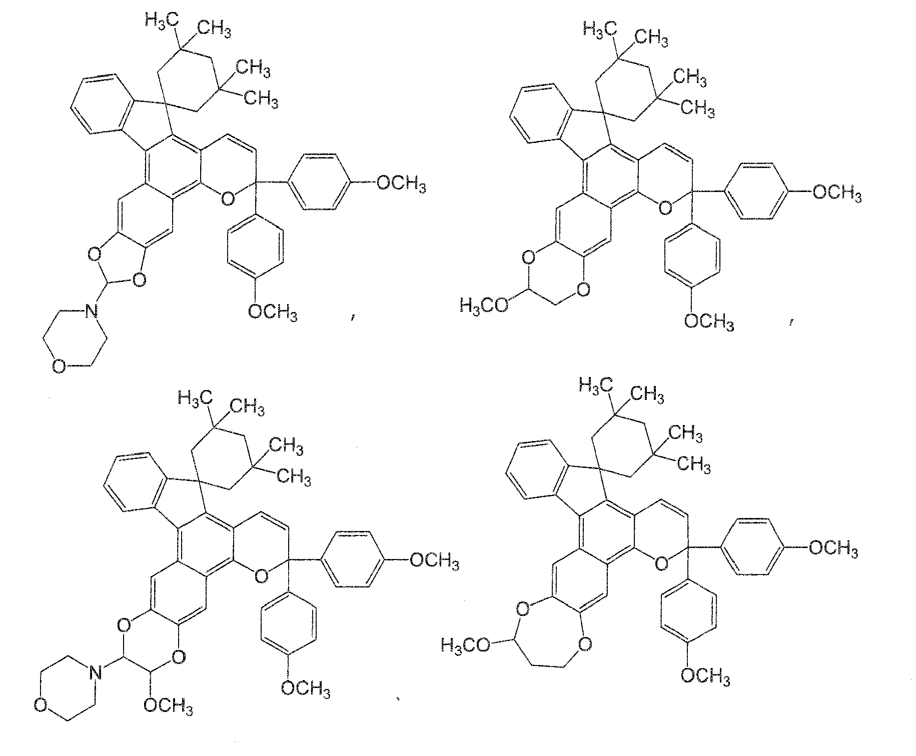

### (identification of chromene compound)

The chromene compound of the present invention is existent as an achromatic or light yellow solid or viscous liquid at normal temperature and normal pressure and can be confirmed by the following means (1) to (3).
(1) When the proton nuclear magnetic resonance spectrum (¹H-NMR) of the chromene compound is measured, a peak based on an aromatic proton appears at δ of around 5.0 to 9.0 ppm and peaks based on the protons of an alkyl group and an alkoxy group appear at δ of around 0.5 to 4.5 ppm. By comparing these spectral intensities relatively, the number of the protons of each bond can be known.
(2) The composition of a corresponding compound can be determined by elemental analysis.
(3) When the ¹³C-nuclear magnetic resonance spectrum (¹³C-NMR) of the chromene compound is measured, a peak based on the carbon of an aromatic hydrocarbon group appears at δ of around 110 to 160 ppm, and peaks based on the carbons of an alkyl group and an alkoxy group appear at δ of around 10 to 80 ppm.

### (production process of chromene compound)

The production process of the chromene compound of the present invention is not particularly limited, and any synthesis process may be employed. As a typical process which is advantageously employed, a naphthol compound and a propargyl alcohol compound are reacted with each other. The production process of the preferred chromene compound represented by the above formula (2) will be described hereinbelow as an example.

The chromene compound represented by the above formula (2) can be advantageously produced by reacting a naphthol compound represented by the following formula (3) with a propargyl alcohol compound represented by the following formula (4) in the presence of an acid catalyst. 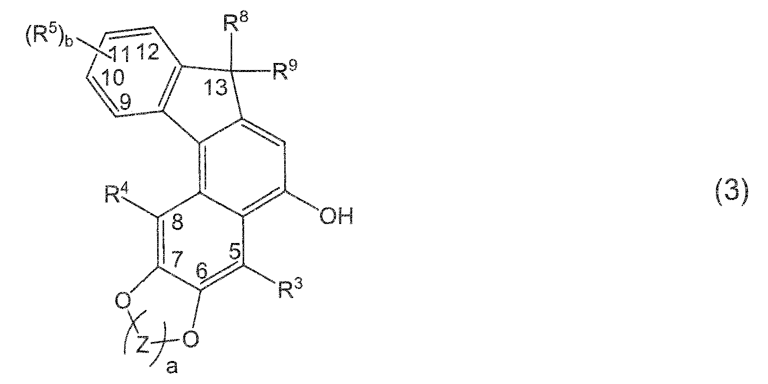 (wherein Z and a are as defined in the above formula (1), and R³, R⁴ R⁵, R⁸ R⁹ and b are as defined in the above formula (2).) 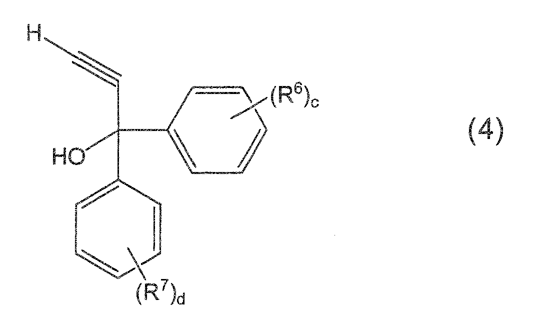 (wherein R⁶, R⁷, c and d are as defined in the above formula (2).)

The naphthol compound represented by the above formula (3) is provided as a novel compound by the present invention.

Examples of Z and "a" in the formula (3) are the same as those in the above formula (1), and examples of R³, R⁴, R⁵, R⁸, R⁹ and "b" are the same as those in the above formula (2). Preferred examples of the naphthol compound represented by the above formula (3) are the following compounds.

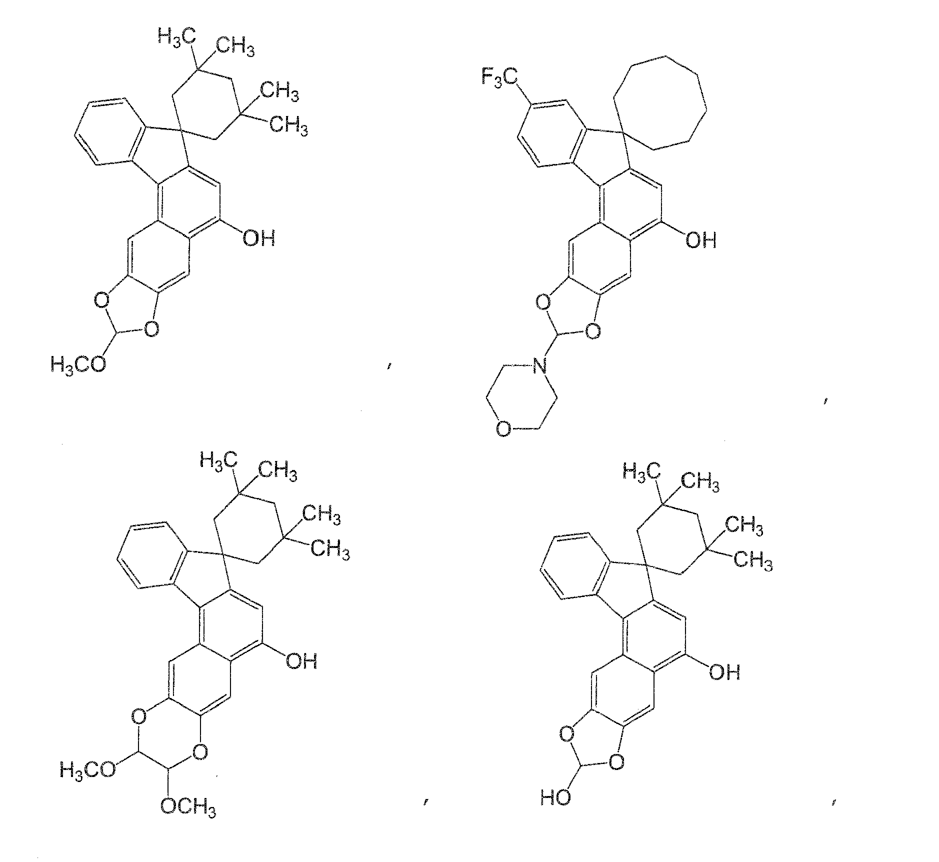

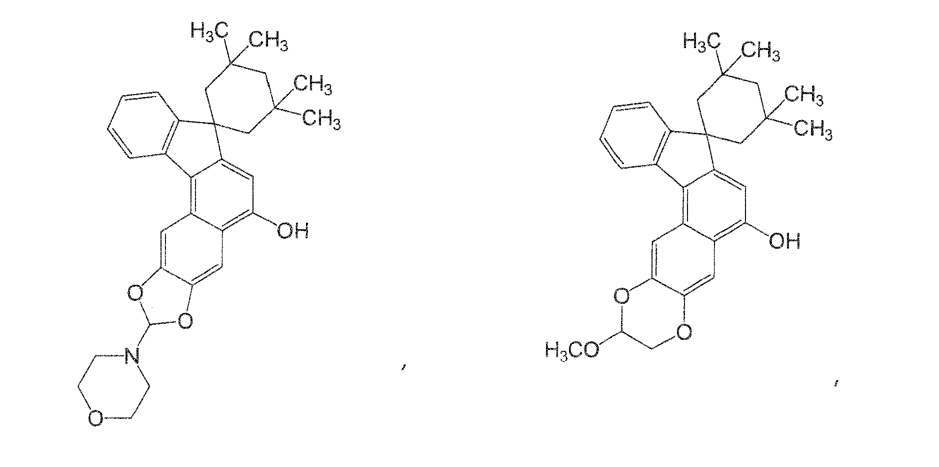

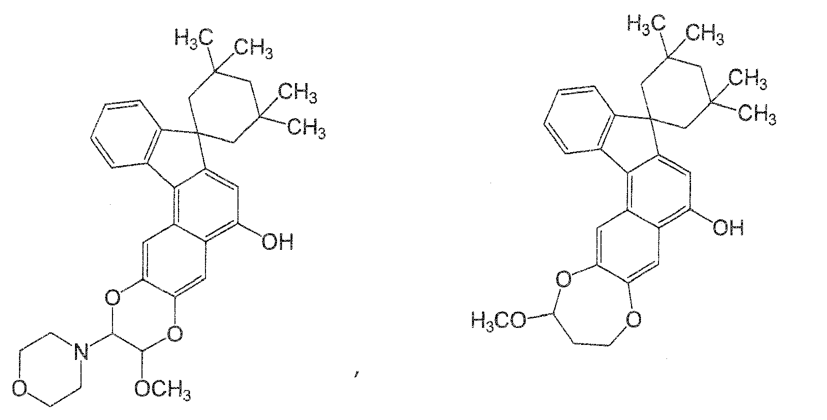

Ordinary naphthol compounds can be synthesized in accordance with a reaction method described in research papers such as Gazzetta Chimica Italiana; 102; 1972; 558-561, Justus Liebigs Annalen der Chemie; 675; 1964; 142-150, Acta Chemica Scandinavia (1947-1973); 10; 1956; 1006-1009, Liebigs Annalen der Chemie; 3; 1982; 507-529, and WO01/60881.

The propargyl alcohol compound represented by the above formula (4) can be synthesized, for example, by reacting a ketone compound corresponding to the above formula (4) with a metal acetylene compound such as lithium acetylide.

### (process of synthesizing naphthol compound)

Although the process of synthesizing the naphthol compound represented by the above formula (3) is not particularly limited, it can be synthesized as follows, for example.

A benzene compound represented by the following formula (5) may be purchased as a commercially available product or may be synthesized based on the following documents. 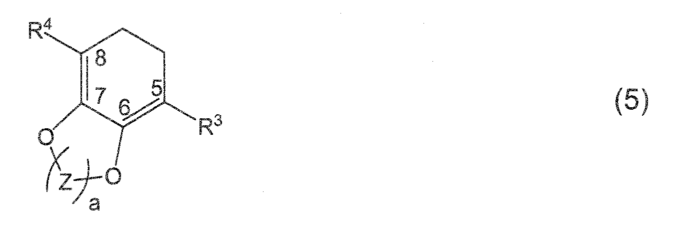 In the above formula, Z and "a" are as defined in the above formula (1) , and R³ and R⁴ are as defined in the above formula (3) .

For example, a benzene compound represented by the following formula (6) can be synthesized in accordance with a reaction method described in research papers such as Izvestiya Vysshikh Uchebnykh Zavedenii, Khimiya i Khimicheskaya Tekhonologiya (1988), 31(5), 46-9., and Synthesis (1985), (1), 31-3.

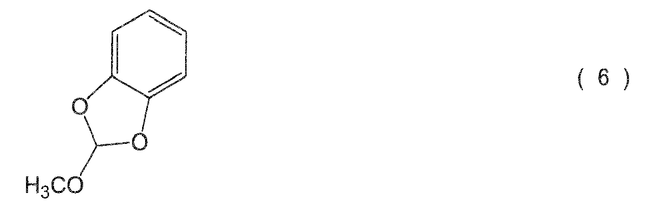

For example, a benzene compound represented by the following formula (7) can be synthesized in accordance with a reaction method described in research papers such as Heterocyclic Communications (2001.), 7(2), 135-141.

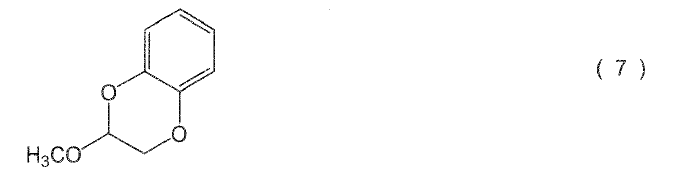

For example, a benzene compound represented by the following formula (8) can be synthesized in accordance with a reaction method described in research papers such as Gazzetta Chemica Italian; English; 102; 1972; 558-561; and ISSN: 0016-5603.

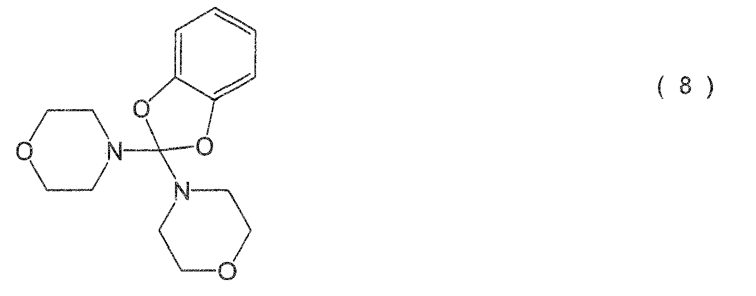

After the obtained benzene compound is brominated, a Grignard reagent is prepared and reacted with acid chloride to obtain a benzophenone compound represented by the following formula (9).

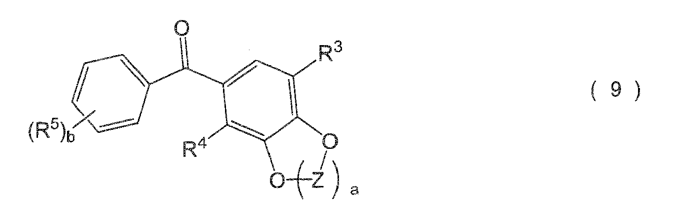

A naphthalene compound represented by the following formula (10) is obtained by carrying out the Stobbe reaction and cyclization reaction of the above benzophenone compound and hydrolyzed by using an alkali or acid to obtain a carboxylic acid represented by the following formula (11).

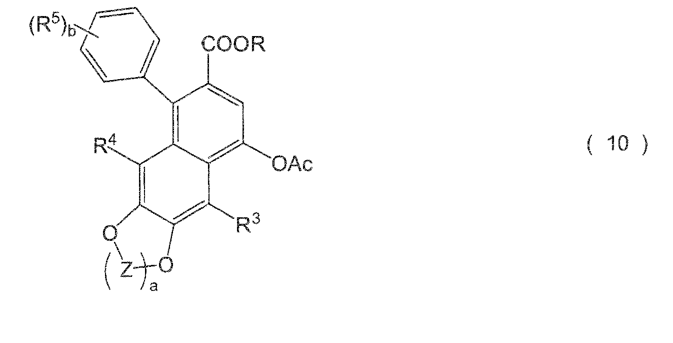

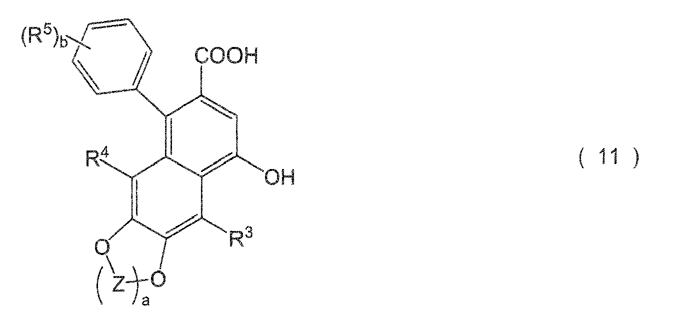

R⁵ and "b" in the above formulas (9) to (11) are as defined in the above formula (3), and R is an alkyl group such as methyl group or ethyl group. The carboxylic acid is benzylated by using a base such as potassium carbonate and benzyl chloride and then hydrolyzed by using an alkali or acid to obtain a carboxylic acid which is benzyl protected and represented by the following formula (12). 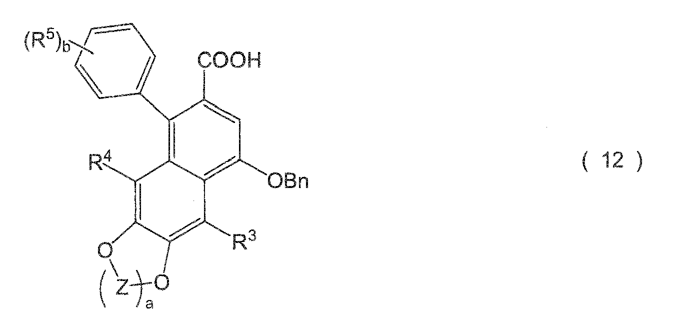 The benzyl protected carboxylic acid is converted into an amine by a method such as Curtius rearrangement, Hofmann rearrangement or Lessen rearrangement, and a diazonium salt is prepared from the amine. This diazonium salt is converted into a bromide through a Sandmeyer reaction or the like, and the obtained bromide is reacted with magnesium or lithium to prepare an organic metal reagent. This organic metal reagent is reacted with a ketone represented by the following formula (13) (R⁸ and R⁹ are as defined in the above formula (3)) at -80 to 70°C in an organic solvent for 10 minutes to 4 hours to obtain an alcohol material represented by the following formula (14). 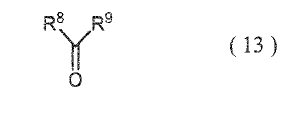 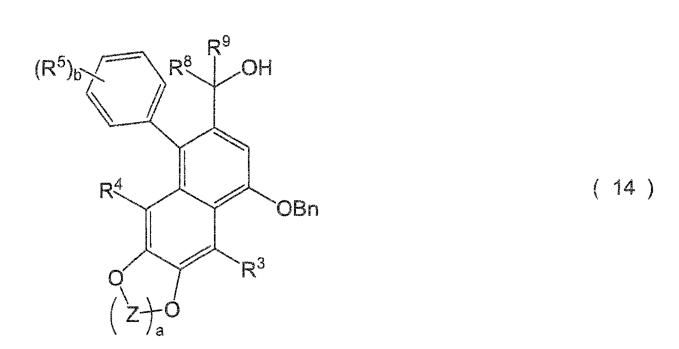 The debenzylation reaction of this alcohol material is carried out with hydrogen and palladium carbon and then a Friedel-Crafts reaction is carried out at 10 to 120°C for 10 minutes to 2 hours under a neutral to acid condition to synthesize a naphthol compound of interest. In the above reaction, the reaction ratio of the above organic metal reagent to the ketone represented by the above formula (13) is selected from among a wide range but generally selected from a range of 1:10 to 10:1 (molar ratio). The reaction temperature is preferably -80 to 70°C, and an aprotic organic solvent such as diethyl ether, tetrahydrofuran, benzene or toluene is used as the solvent. The naphthol compound represented by the above formula (3) can be obtained by carrying out the Friedel-Crafts reaction of the alcohol material in the neutral to acid condition. The acid catalyst is preferably selected from acetic acid, hydrochloric acrid, sulfuric acid, benzenesulfonic acid, p-toluenesulfonic acid and acid alumina. The acid catalyst is preferably used in an amount of 0.1 to 10 parts by weight based on 100 parts by weight of the alcohol material. For this reaction, a solvent such as tetrahydrofuran, benzene or toluene is used.

The reaction ratio of the naphthol compound to the propargyl alcohol compound is preferably selected from a range of 1:10 to 10:1 (molar ratio). The acid catalyst is preferably selected from sulfuric acid, benzenesulfonic acid, p-toluenesulfonic acid and acid alumina. The acid catalyst is preferably used in an amount of 0.1 to 10 parts by weight based on 100 parts by weight of the total of the naphthol compound and the propargyl alcohol compound. The reaction temperature is preferably 0 to 200°C, and an aprotic organic solvent such as N-methylpyrrolidone, dimethyl formamide, tetrahydrofuran, benzene or toluene is used as the solvent. The method of purifying the product obtained by the above reaction is not particularly limited. For example, the product is subjected to silica gel column purification and further purified by re-crystallization.

### (characteristic properties of chromene compound)

Since the chromene compound of the present invention has high double peak characteristic, when it is mixed with another photochromic compound to prepare a photochromic composition which develops a brown or gray color, the amount of the photochromic compound which develops a yellow color and has low durability can be reduced. Therefore, a color change at the time of fading and a color change at the time of deterioration hardly occur. Further, since the chromene compound of the present invention has little initial coloration, an optical article containing the chromene compound of the present invention, for example, a photochromic lens containing the chromene compound of the present invention has high transparency under no exposure to light.

As the photochromic compound to be mixed with the chromene compound of the present invention so as to adjust the color may be used a known compound. Examples of this photochromic compound include chromene compounds described in a pamphlet of International Laid-open W02001/060811 and JP-A 2009-67680.

### (use of chromene compound)

The chromene compound of the present invention exhibits excellent photochromic properties as described above. It is most practical to disperse the chromene compound of the present invention into a polymer material, and a photochromic optical article having a polymer molded product containing the chromene compound of the present invention dispersed therein as a constituent member exhibits excellent photochromic properties. Therefore, the chromene compound of the present invention can be used especially in photochromic lenses which are optical articles.

When the chromene compound of the present invention is used in a photochromic lens, a lens can be formed by any commonly used method as long as uniform light controllability is obtained. For example, a method in which a thermoplastic resin and the chromene compound of the present invention are mixed together in a molten state to form a lens is employed. Further, a method in which a polymer film containing the chromene compound of the present invention dispersed uniformly therein is formed on the surface of a lens, or a method in which the chromene compound of the present invention is dissolved, for example, in silicone oil and impregnated into the surface of a lens at 150 to 200°C for 10 to 60 minutes is also employed. When the chromene compound is dispersed into the surface portion of the lens as described above, the surface of the lens may be further coated with a curable substance as required, to obtain a photochromic lens.

Moreover, a method in which a photochromic curable composition containing the chromene compound of the present invention and a polymerizable monomer is polymerized by a predetermined method to obtain a lens may be employed. In the method using this photochromic curable composition, a photochromic lens can be formed directly by polymerizing the curable composition by a known method. Further, the photochromic curable composition may be applied to a plastic lens (optical substrate) and polymerized and cured to form a polymer film containing the chromene compound of the present invention dispersed therein on the optical substrate, thereby obtaining a photochromic lens (this method may be referred to as "coating method"). When the polymer film containing the chromene compound of the present invention dispersed therein is formed on the optical substrate, the surface of the polymer film may be further coated with a curable substance.

In the above photochromic curable composition, the polymerizable monomer in use may be a known polymerizable monomer, and a combination of known polymerizable monomers may be selected according to the desired performance of an optical article.

### Examples

The following examples are provided for the purpose of further illustrating the present invention but are in no way to be taken as limiting.

### Example 1

1.00 g (2.33 mmol) of a naphthol compound represented by the following formula (15) and 0.92 g (3.43 mmol) of a propargyl alcohol compound represented by the following formula (16) were dissolved in 50 ml of toluene, 0.022 g of p-toluenesulfonic acid was further added to the resulting solution, and the obtained mixture was refluxed for 1 hour. 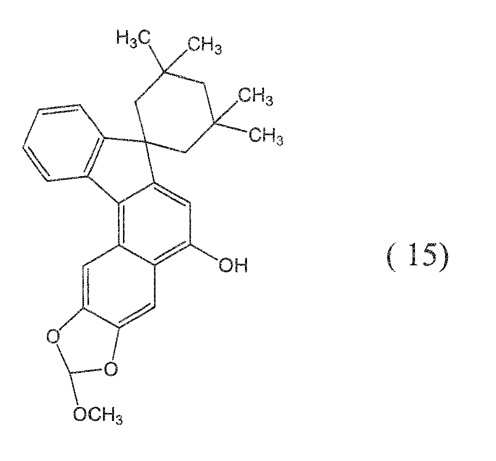 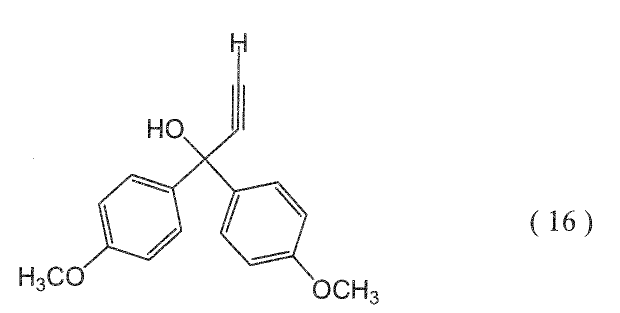 After the reaction, the solvent was removed, the obtained product was purified by column chromatography, and crystallization was carried out with methanol (5 ml) to obtain 1.11 g of a white powder (yield rate of 70 %). The elemental analysis values of this product were 79.44 % of C, 6.49 % of H and 0.00 % of N which were almost equal to the calculated values of C₄₅H₄₄O₆ (C: 79.39 %, H: 6.51 %, N: 0.00 %).

When the proton nuclear magnetic resonance spectrum of the product was measured, it showed 28H peaks based on an alkyl group and an alkoxy group at δ of around 0.5 to 4.5 ppm and a 16H peak based on an aromatic proton at δ of around 5.0 to 9.0 ppm.

Further, when the ¹³C-nuclear magnetic resonance spectrum was measured, it showed a peak based on the carbon of an aromatic ring at δ of aground 110 to 160 ppm and peaks based on the carbons of an alkyl group and an alkoxy group at δ of around 10 to 80 ppm.

It was confirmed from the above results that the isolated product was a compound represented by the following formula (17).

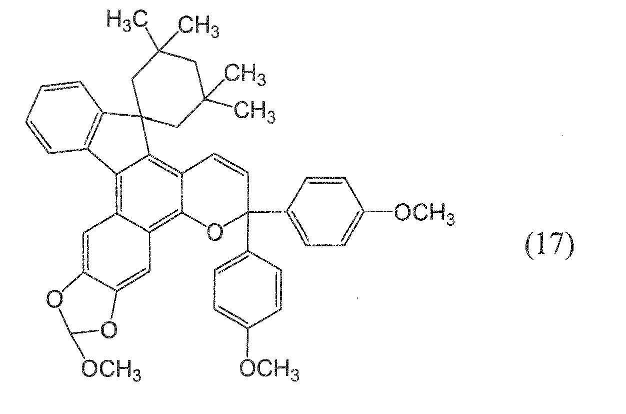

### Examples 2 and 3

Chromene compounds shown in Table 1 were synthesized in the same manner as in Example 1. When the structures of the obtained products were analyzed by using the same structure confirming means as in Example 1, it was confirmed that they were compounds represented by structural formulas shown in Table 1. The elemental analysis values, calculated values obtained from the structural formulas of the compounds and characteristic ¹H-NMR spectra of these compounds are shown in Table 2.

**Table 1**

| Example No. | Raw materials | | Product | Yield rate (%) |
|---|---|---|---|---|
| | Naphthol derivative | Propargyl alcohol derivative | | |
| 2 | 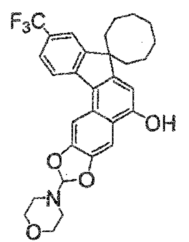 | 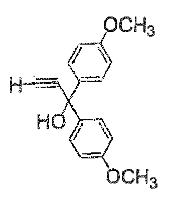 | 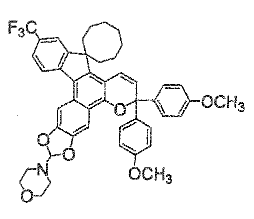 | 65 |
| 3 | 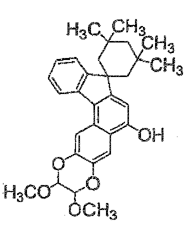 | 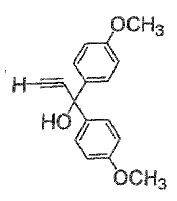 | 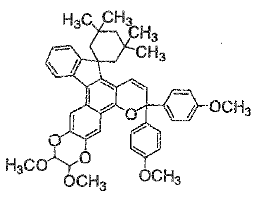 | 67 |

**Table 2**

| Compound No. | Elemental analysis values | | | | | | ¹H-NMR (NMR) |
|---|---|---|---|---|---|---|---|
| | Experimental values | | | Calculated values | | | |
| | C | H | N | C | H | N | |
| 2 | 72.66 | 5.77 | 1.80 | 72.76 | 5.72 | 1.81 | δ5.0-9.0 15H δ0.5-4.5 29H |
| 3 | 77.89 | 6.77 | 0.00 | 77.88 | 6.67 | 0.00 | δ5.0-9.0 16H δ0.5-4.5 32H |

### Example 4

### (evaluation of physical properties of photochromic plastic lens produced by coating method)

The chromene compound obtained in Examples 1 was mixed with a photopolymerization initiator and polymerizable monomers, the resulting mixture was applied to the surface of a lens substrate, and ultraviolet light was applied to polymerize the coating film on the surface of the lens substrate.

For a photochromic curable composition, a mixture of 50 parts by mass of
2,2-bis(4-methacryloyloxypentaethoxyphenyl)propane, 10 parts by mass of polyethylene glycol diacrylate (average molecular weight of 532), 10 parts by mass of trimethylolpropane trimethacrylate, 10 parts by mass of polyester oligomer hexaacrylate (EB-1830 of Daicel UCB Co., Ltd.) and 10 parts by mass of glycidyl methacrylate as radical polymerizable monomers was used. 1 part by mass of the Chromene compound obtained in Example 1 was added to and fully mixed with 90 parts by mass of this mixture of the radical polymerizable monomers, and 0.3 part by mass of CGI1800 {a mixture of 1-hydroxycyclohexylphenyl ketone and bis(2,6-dimethoxybenzoyl)-2,4,4-trimethyl-pentyl phosphine oxide (weight ratio of 3:1)} as a photopolymerization initiator, 5 parts by mass of bis (1,2,2,6,6-pentamethyl-4-piperidyl)sebacate, 3 parts by mass of ethylenebis(oxyethylene)bis[3-(5-tert-butyl-4-hydroxy-m-tolyl)propionate] as a stabilizer, 7 parts by mass of γ-methacryloyloxypropyl trimethoxysilane as a silane coupling agent and 3 parts by mass of N-methyldiethanolamine were added to and fully mixed with the above mixture to obtain a photochromic curable composition.

Subsequently, about 2 g of the photochromic curable composition obtained by the above method was applied to the surface of a lens substrate (CR39: acrylic resin plastic lens; refractive index of 1.50) by using the 1H-DX2 spin coater of MIKASA Co., Ltd. This coated lens was irradiated with light from a metal halide lamp having an output of 120 mW/cm² in a nitrogen gas atmosphere for 3 minutes to cure the photochromic curable composition. Thus, an optical article (photochromic plastic lens) which was coated with a polymer film containing the chromene compound dispersed therein (thickness of polymer film: 40 µm) was manufactured.

The following Photochromic properties of the obtained photochromic plastic lens were evaluated. The results are shown in Table 3.
[1] Maximum absorption wavelength (λmax): This is the maximum absorption wavelength after color development obtained by means of the spectrophotometer (MCPD3000 instantaneous multi-channel photodetector) of Otsuka Electronics Co., Ltd. and used as an index of color at the time of color development. The maximum absorption wavelength is connected with color at the time of color development.
[2] Color optical density (A₀): This is the difference between absorbance {ε(120) after 120 seconds of exposure and ε(0) under no exposure at the above maximum absorption wavelength and used as an index of color optical density. It can be said that as this value becomes larger, the photochromic properties become better.
[3] Double peak characteristic (A_{Y}/A_{B}): This is the ratio of color optical density (A_{Y}: value of λₘₐₓ) at a yellow range (having a maximum absorption wavelength at 430 to 530 nm) and color optical density (A_{B}: value of λₘₐₓ) at a blue range (having a maximum absorption wavelength at 550 to 650 nm) and used as an index of double peak characteristic.
[4] Fading half period [τ1/2(sec.)]: time required for the reduction of the absorbance at the above maximum absorption wavelength of a sample to 1/2 of {ε(120) -ε(0)} when exposure is stopped after 120 seconds of exposure and used as an index of fading speed. As this time becomes shorter, the fading speed becomes higher.
[5] Absorption end {λ₀}: After the photochromic plastic lens obtained under the above conditions is used as a sample and kept in the dark for one day, the ultraviolet light transmittance (T%) at 300 to 800 nm of the sample is measured with an ultraviolet visible spectrophotometer (UV-2550 of Shimadzu Corporation) at room temperature. A tangent line is drawn on the obtained ultraviolet light absorption curve to ensure that the transmittance (T%) of the ultraviolet light absorption curve passes a point of 50 % so as to obtain an absorption wavelength at which the transmittance (T%) of the tangent line becomes 0 as the absorption end (absorption end of the ultraviolet light spectrum) and used as an index of initial coloration. For example, in an optical article such as a spectacle lens, as this value becomes smaller, the initial coloration becomes weaker and the transparency under no exposure becomes higher.
[6] Thermochromism {T₀}: The photochromic plastic lens obtained under the above conditions is used as a sample and its transmittance (T%) at 300 to 800 nm is measured with an ultraviolet visible spectrophotometer (UV-2550 of Shimadzu Corporation) at room temperature. This is a transmittance at a wavelength at which the transmittance at 430 to 650 nm becomes minimal and used as an index of initial coloration. As this value becomes larger, the initial coloration becomes weaker and the transparency under no exposure becomes higher.
[7] Residual rate (A₅₀/A₀ x 100): The deterioration promotion test of the obtained photochromic plastic lens is carried out by using the X25 xenon weather meter of Suga Test Instruments Co., Ltd. for 50 hours. Thereafter, the above color optical density is evaluated before and after the test, the color optical density (A₀) before the test and the color optical density (A₅₀) after the test are measured, and the ratio (A₅₀/A₀) of these values is taken as residual rate and used as an index of color development durability. As the residual rate becomes higher, the color development durability becomes higher.

### Examples 5 and 6

The characteristic properties of photochromic plastic lenses were evaluated in the same manner as in Example 4 except that the compounds obtained in Examples 2 and 3 were used as the Chromene compounds. The results are shown in Table 3. In Table 3, the compound Nos. 1 to 3 are chromene compounds obtained in Examples 1 to 3, respectively.

**Table 3**

| Example No. | Compound No. | λmax | Color optical density | Double peak characteristic | Fading half period | Initial coloration (absorption end) | Initial coloration (thermochromism) | Residual rate |
|---|---|---|---|---|---|---|---|---|
| | | (nm) | A₀ | A_{Y} /A_{B} | τ1/2 (sec) | (nm) | (%) | (A₅₀/A₀) X100 |
| 4 | 1 | 446 567 | 0.35 0.28 | 1.25 | 62 59 | 405 | 87 87 | 88 89 |
| 5 | 2 | 452 575 | 0.40 0.32 | 1.25 | 89 88 | 405 | 84 84 | 83 84 |
| 6 | 3 | 448 569 | 0.36 0.29 | 1.24 | 65 65 | 405 | 87 87 | 88 88 |

### Comparative Examples 1 to 5

For comparison, the operation of Example 4 was repeated by using the compound of the following formulas (A) (Comparative Example 1), the compound of the following formula (B) (Comparative Example 2), the compound of the following formula (C) (Comparative Example 3), the compound of the following formula (D) (Comparative Example 4) and the compound of the following formula (E) (Comparative Example 5). The chromene compounds used in these comparative examples are given below.

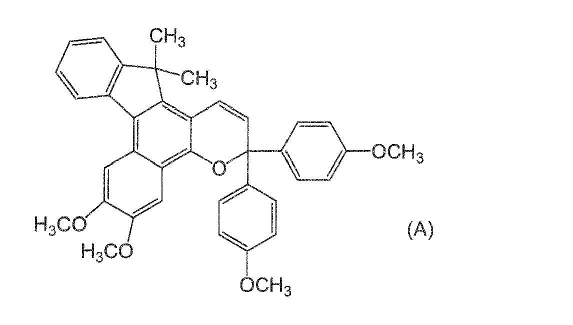

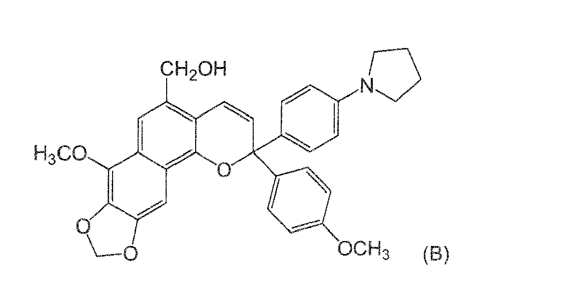

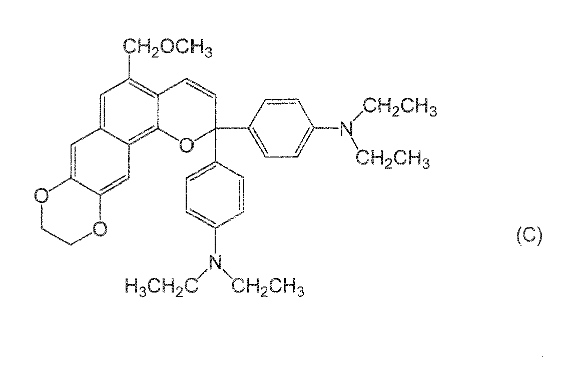

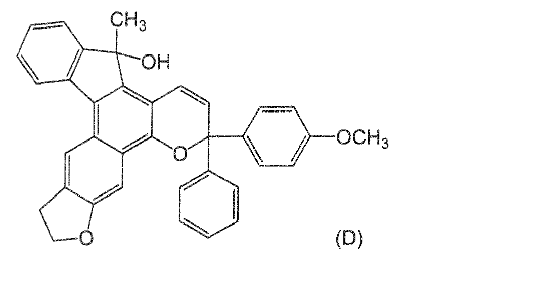

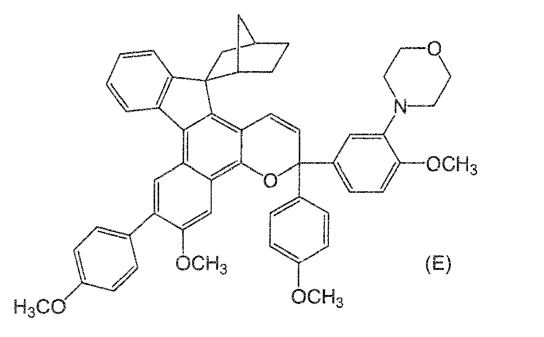

Photochromic plastic lenses were obtained by using the above chromene compounds and their photochromic properties were evaluated in the same manner as in Example 4. The results are shown in Table 4.

**Table 4**

| Example No. | Compound No. | λmax | Color optical density | Double peak characteristic | Fading half period | Initial coloration (absorption end) | Initial coloration (thermochromism) | Residual rate |
|---|---|---|---|---|---|---|---|---|
| | | (nm) | A₀ | A_{Y} /A_{B} | τ1/2 (sex) | (nm) | (%) | (A_{50/}A₀)X100 |
| 1 | A | 457 574 | 0.69 0.45 | 1.56 | 195 196 | 397 | 67 75 | 76 77 |
| 2 | B | 475 585 | 0.26 0.32 | 0.80 | 140 140 | 404 | 77 77 | 69 69 |
| 3 | C | 475 593 | 0.19 0.33 | 0.53 | 82 82 | 404 | 68 65 | 65 65 |
| 4 | D | 455 576 | 0.30 0.32 | 0.94 | 83 83 | 410 | 77 78 | 35 35 |
| 5 | E | 458 568 | 0.44 0.37 | 1.20 | 68 68 | 422 | 84 86 | 85 84 |

In Comparative Example 1, although the color optical density and double peak characteristic of the lens are satisfactory and the initial coloration is little, the fading speed is low. In Comparative Examples 2, 3 and 4, the double peak characteristic is low, which is not preferred in terms of color control when a photochromic plastic lens developing a color of a neutral tint is to be manufactured. In Comparative Example 5, although the color optical density and double peak characteristic of the lens are satisfactory, the initial coloration is large as the absorption end is existent at a visible range.

In contrast to this, in Examples 4 to 6 in which the chromene compound of the present invention is used, as compared with Comparative Example 1, the fading speed is high. As compared with Comparative Examples 2, 3 and 4, the double peak characteristic is high. Since the absorption end is at a short wavelength range as compared with Comparative Example 5, the initial coloration is little. Further, the chromene compounds of the present invention are all satisfactory in terms of durability.

Since the chromene compound of the present invention develops a color of a neutral tint by itself, it can be used alone and hardly undergoes a color change at the time of fading and a color change at the time of deterioration. Further, since the chromene compound has little initial coloration, high color optical density, high double peak characteristic and high fading speed, an extremely excellent photochromic lens can be obtained from the chromene compound. Therefore, color can be controlled by mixing it with another Photochromic compound, and even when it is mixed with another photochromic compound, it can exhibit excellent photochromic properties.

### Examples 7 to 25

Chromene compounds shown in Table 5 were synthesized in the same manner as in Example 1. When the structures of the obtained chromene compounds were analyzed in the same manner as in Example 1, it was confirmed that they were compounds represented by the structural formulas shown in Table 5. Table 6 shows the elemental analysis values and ¹H-NMR spectral values of the chromene compounds obtained in these Examples. In Table 6, the compound Nos. 7 to 25 are chromene compounds obtained in Examples 7 to 25, respectively.

**Table 5**

| Example No. | Raw materials | | Product | Yield rate (%) |
|---|---|---|---|---|
| | Naphthol compound | propargyl alcohol compound | | |
| 7 | 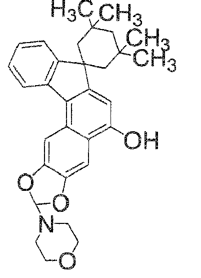 | 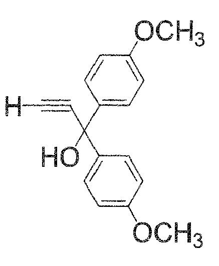 | 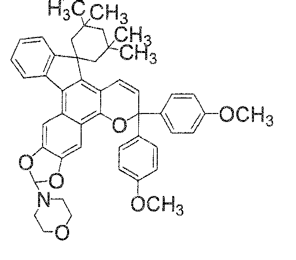 | 67 |
| 8 | 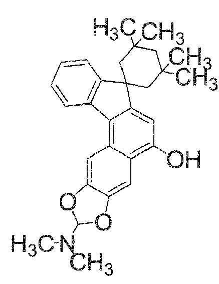 | 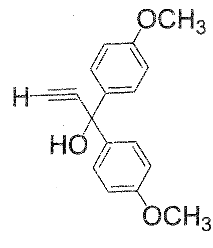 | 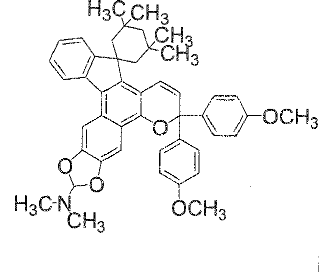 | 69 |
| 9 | 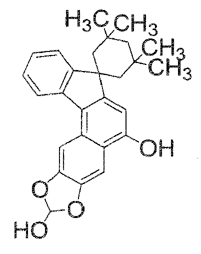 | 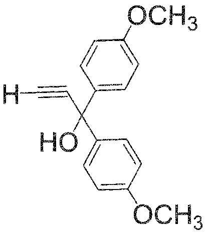 | 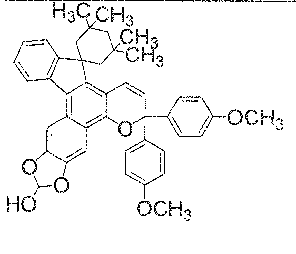 | 60 |
| 10 | 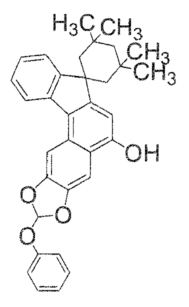 | 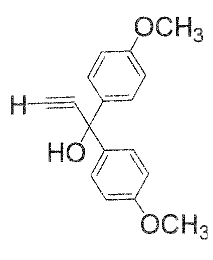 | 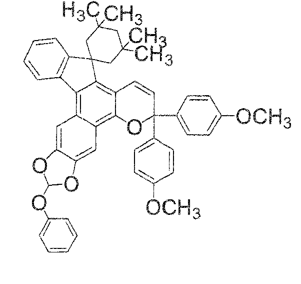 | 67 |
| 11 | 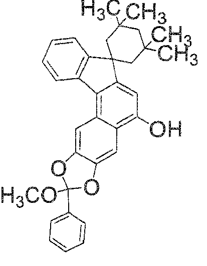 | 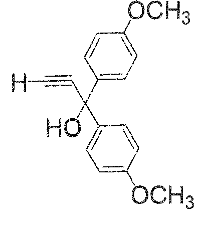 | 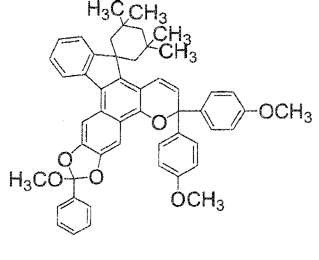 | 67 |
| 12 | 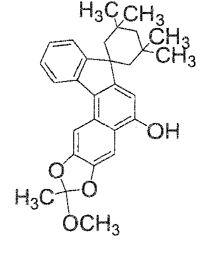 | 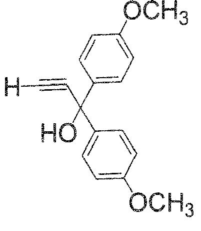 | 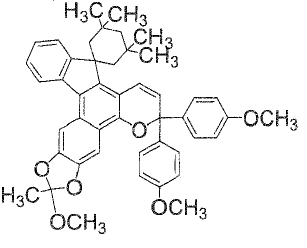 | 66 |
| 13 | 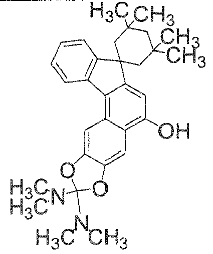 | 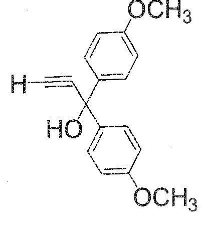 | 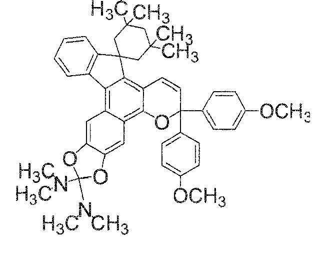 | 65 |
| 14 | 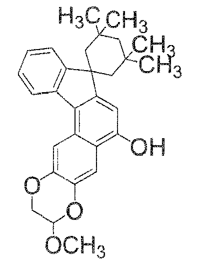 | 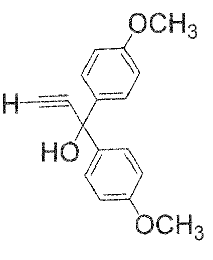 | 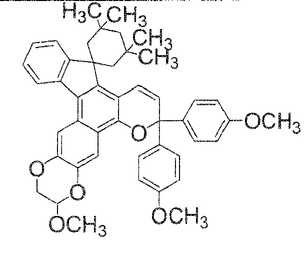 | 65 |
| 15 | 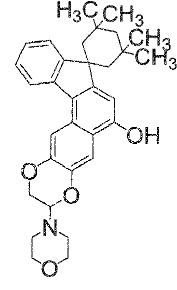 | 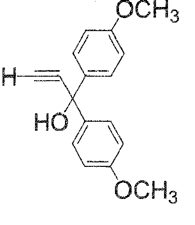 | 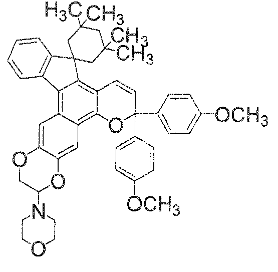 | 66 |
| 16 | 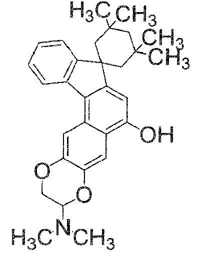 | 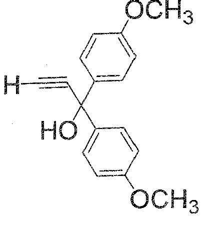 | 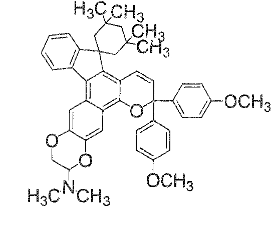 | 65 |
| 17 | 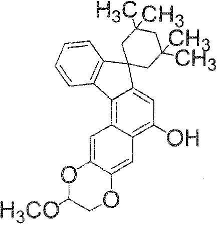 | 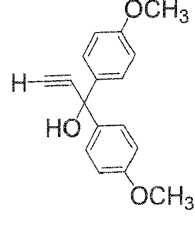 | 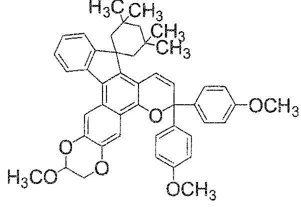 | 65 |
| 18 | 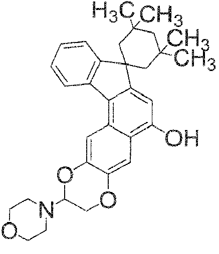 | 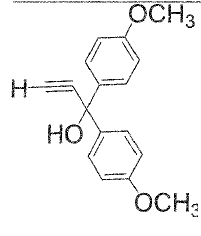 | 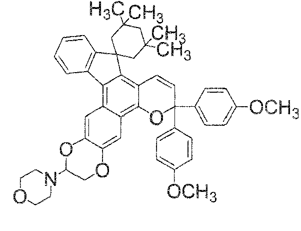 | 65 |
| 19 | 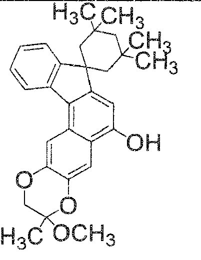 | 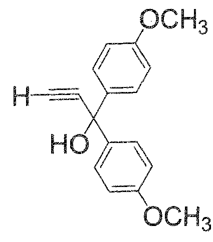 | 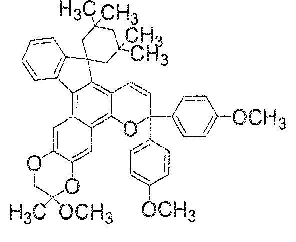 | 62 |
| 20 | 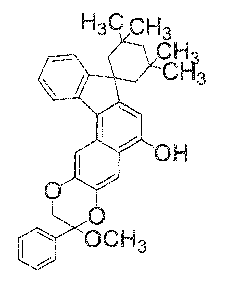 | 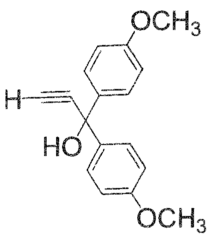 | 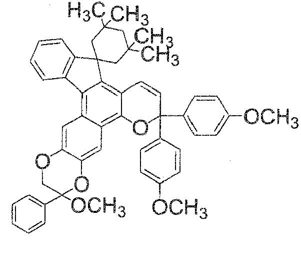 | 63 |
| 21 | 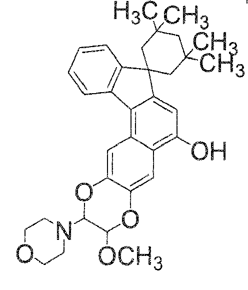 | 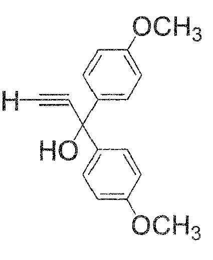 | 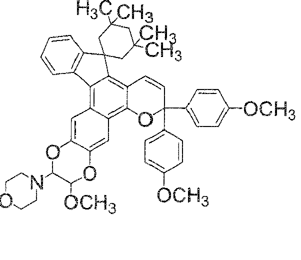 | 62 |
| 22 | 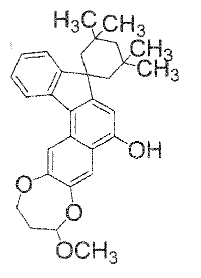 | 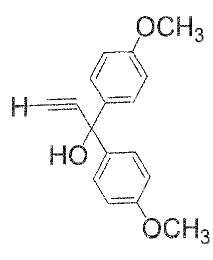 | 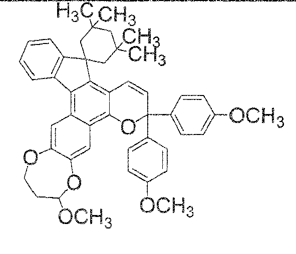 | 61 |
| 23 | 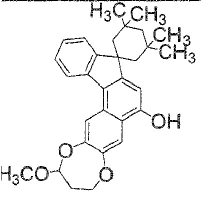 | 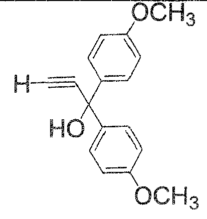 | 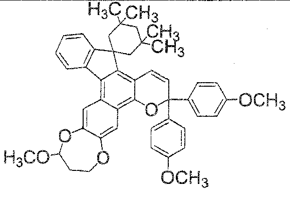 | 62 |
| 24 | 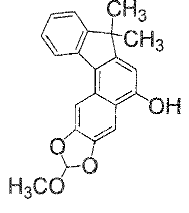 | 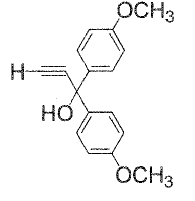 | 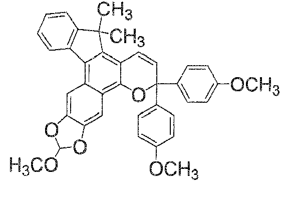 | 68 |
| 25 | 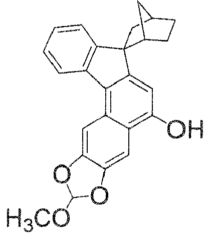 | 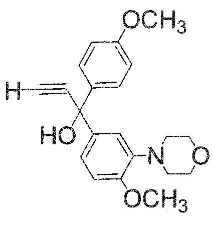 | 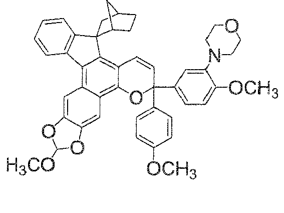 | 64 |

**Table 6**

| Compound No. | Elemental analysis values | | | | | | ¹H-NMR (NMR) |
|---|---|---|---|---|---|---|---|
| | Experimental values | | | Calculated values | | | |
| | C | H | N | C | H | N | |
| 7 | 78.39 | 6.64 | 1.92 | 78.34 | 6.71 | 1.90 | δ5.0-9.0 16H δ0.5-4.5 33H |
| 8 | 79.74 | 6.79 | 2.00 | 79.62 | 6.83 | 2.02 | δ5.0-9.0 16H δ0.5-4.5 31H |
| 9 | 79.32 | 6.37 | 0.00 | 79.25 | 6.35 | 0.00 | δ5.0-9.0 16H δ0.5-4.5 26H |
| 10 | 80.80 | 6.21 | 0.00 | 80.84 | 6.24 | 0.00 | δ5.5-9.0 21H δ0.5-4.5 25H |
| 11 | 80.90 | 6.45 | 0.00 | 80.93 | 6.39 | 0.00 | δ5.0-9.0 21H δ0.5-4.5 27H |
| 12 | 79.44 | 6.72 | 0.00 | 79.51 | 6.67 | 0.00 | δ5.0-9.0 16H δ0.5-4.5 30H |
| 13 | 78.22 | 7.09 | 3.83 | 78.23 | 7.11 | 3.80 | δ5.0-9.0 16H δ0.5-4.5 36H |
| 14 | 79.55 | 6.73 | 0.00 | 79.51 | 6.67 | 0.00 | δ5.0-9.0 16H δ0.5-4.5 30H |
| 15 | 78.56 | 6.79 | 1.90 | 78.48 | 6.85 | 1.87 | δ5.0-9.0 16H δ0.5-4.5 35H |
| 16 | 79.76 | 6.99 | 2.00 | 79.74 | 6.98 | 1.98 | δ5.0-9.0 16H δ0.5-4.5 33H |
| 17 | 79.48 | 6.66 | 0.00 | 79.51 | 6.67 | 0.00 | δ5.0-9.0 16H δ0.5-4.5 30H |
| 18 | 78.41 | 6.90 | 1.85 | 78.48 | 6.85 | 1.87 | δ5.0-9.0 16H δ0.5-4.5 35H |
| 19 | 79.66 | 6.82 | 0.00 | 79.63 | 6.83 | 0.00 | δ5.0-9.0 16H δ0.5-4.5 32H |
| 20 | 81.00 | 6.58 | 0.00 | 81.01 | 6.54 | 0.00 | δ5.0-9.0 21H δ0.5-4.5 29H |
| 21 | 77.04 | 6.79 | 1.81 | 77.00 | 6.85 | 1.80 | δ5.0-9.0 16H δ0.5-4.5 37H |
| 22 | 79.61 | 6.88 | 0.00 | 79.63 | 6.83 | 0.00 | δ5.0-9.0 16H δ0.5-4.5 32H |
| 23 | 79.56 | 6.83 | 0.00 | 79.63 | 6.83 | 0.00 | δ5.0-9.0 16H δ0.5-4.5 32H |
| 24 | 78.00 | 5.58 | 0.00 | 78.06 | 5.52 | 0.00 | δ5.0-9.0 16H δ0.5-4.5 16H |
| 25 | 76.55 | 6.02 | 1.93 | 76.54 | 0.00 | 1.94 | δ5.0-9.0 15H δ0.5-4.5 28H |

### Examples 26 to 44

Photochromic plastic lenses were manufactured and their characteristic properties were evaluated in the same manner as in Example 4 except that the compounds obtained in Examples 7 to 25 were used as chromene compounds. The results are shown in Table 7. In Table 7, the compound Nos. 7 to 25 are chromene compounds obtained in Examples 7 to 25, respectively.

**Table 7**

| Example No. | Compound No. | λmax | Color optical density | Double peak characteristic | Fading half period | Initial coloration (absorption end) | Initial coloration (thermochromism) | Residual rate |
|---|---|---|---|---|---|---|---|---|
| | | (nm) | A₀ | A_{Y}/A_{B} (sec) | τ1/2 | (nm) | (%) | (A₅₀/A₀) x100 |
| 26 | 7 | 453 576 | 0.56 0.40 | 1.40 | 86 86 | 410 | 85 85 | 86 86 |
| 27 | 8 | 458 581 | 0.82 0.51 | 1.61 | 129 129 | 413 | 82 82 | 84 84 |
| 28 | 9 | 450 571 | 0.48 0.35 | 1.37 | 86 86 | 408 | 86 86 | 80 80 |
| 29 | 10 | 448 569 | 0.45 0.35 | 1.29 | 80 80 | 407 | 87 87 | 86 86 |
| 30 | 11 | 445 567 | 0.35 0.30 | 1.17 | 65 65 | 410 | 85 85 | 85 85 |
| 31 | 12 | 449 570 | 0.48 0.35 | 1.37 | 85 85 | 407 | 86 86 | 86 86 |
| 32 | 13 | 468 587 | 0.95 0.51 | 1.86 | 145 145 | 418 | 80 80 | 82 82 |
| 33 | 14 | 446 567 | 0.33 0.29 | 1.14 | 64 64 | 405 | 87 87 | 87 87 |
| 34 | 15 | 454 576 | 0.54 0.39 | 1.38 | 95 95 | 411 | 85 85 | 86 86 |
| 35 | 16 | 459 580 | 0.84 0.50 | 1.68 | 136 136 | 413 | 82 82 | 84 84 |
| 36 | 17 | 447 567 | 0.35 0.29 | 1.21 | 65 65 | 404 | 87 87 | 87 87 |
| 37 | 18 | 455 577 | 0_{.}57 0.39 | 1.46 | 98 98 | 411 | 84 84 | 86 86 |
| 38 | 19 | 450 579 | 0.49 0.34 | 1.44 | 87 87 | 408 | 86 86 | 86 86 |
| 39 | 20 | 450 581 | 0.36 0.29 | 1.24 | 66 66 | 410 | 85 85 | 85 85 |
| 40 | 21 | 455 580 | 0.65 0.42 | 1.55 | 110 111 | 412 | 84 84 | 85 85 |
| 41 | 22 | 446 566 | 0.31 0.27 | 1.15 | 70 70 | 405 | 86 86 | 85 85 |
| 42 | 23 | 447 567 | 0.33 0.27 | 1.22 | 71 71 | 405 | 86 86 | 85 85 |
| 43 | 24 | 447 565 | 0.52 0.41 | 1.27 | 159 159 | 405 | 80 80 | 85 85 |
| 44 | 25 | 449 567 | 0.45 0.32 | 1.41 | 81 81 | 407 | 84 85 | 85 84 |

Examples of the naphthol compound are given below.

### Example 45

27.2 g (178.5 mmol) of a benzene derivative represented by the following formula (18) and 14 g of Wakogel C-300 (of Wako Pure Chemical Industries, Ltd.) were dissolved in 2, 000 ml of dichloromethane, the resulting solution was cooled to - 15°C, and 31.3 g (174.9 mmol) of N-bromosuccinimide was added and stirred for 12 hours. After the reaction, the reaction product was washed in water, the solvent was removed, and the obtained product was purified by column chromatography to obtain a compound represented by the following formula (19) as 39.2 g (169.6 mmol, yield rate of 95 %) of orange oil.

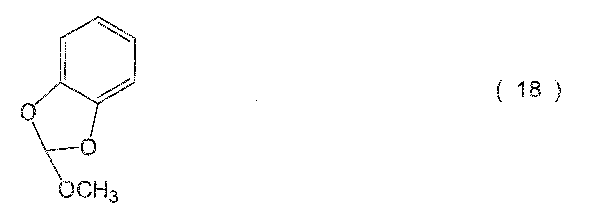

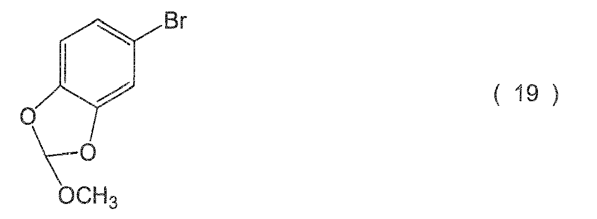

4.5 g (185.2 mmol) of magnesium was added to 200 ml of tetrahydrofuran and heated to 55°C. A tetrahydrofuran (200 ml) solution, of the compound of the above formula (19) was added dropwise to the above solution to prepare a Grignard reagent. The obtained Grignard reagent was cooled to -78°C, and a tetrahydrofuran (200 ml) solution of 26. 0 g (185.0 mmol) of benzoyl chloride was added dropwise to the reagent. After the addition, the resulting solution was heated up to room temperature and stirred for 3 hours. After the reaction, toluene was added, the reaction product was washed in water, the solvent was removed, and the obtained product was purified by recrystallization with methanol to obtain a compound represented by the following formula (20) as 31.7 g (123.8 mmol, yield rate of 73 %) of a yellow solid.

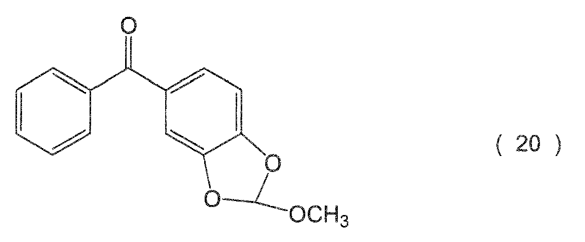

The compound of the above formula (20) and 24.8 g (142.4 mmol) of diethyl succinate were dissolved in 250 ml of tetrahydrofuran and heated to 55°C. A tetrahydrofuran solution (250 ml) of 16.0 g (142.4 mmol) of potassium-t-butoxide was added dropwise to this solution and stirred for 1 hour. After the reaction, toluene was added, the reaction product was washed with concentrated hydrochloric acid and then with water, and the solvent was removed to obtain a compound represented by the following formula (21) as 47.6 g (123.8 mmol, yield rate of 100 %) of orange oil.

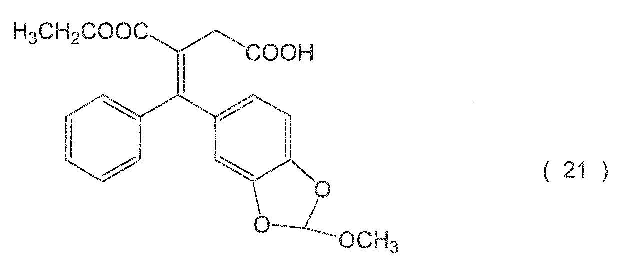

The compound of the above formula (21), 11.2 g (136.2 mmol) of sodium acetate and 63.2 g (619.0 mmol) of acetic anhydride were dissolved in 180 ml of toluene and refluxed for 3 hours. After the reaction, the reaction product was washed in water, the solvent was removed, and the obtained product was purified by recrystallization with methanol to obtain a compound represented by the following formula (22) as 15.2 g (37.1 mmol, yield rate of 30 %) of an orange solid.

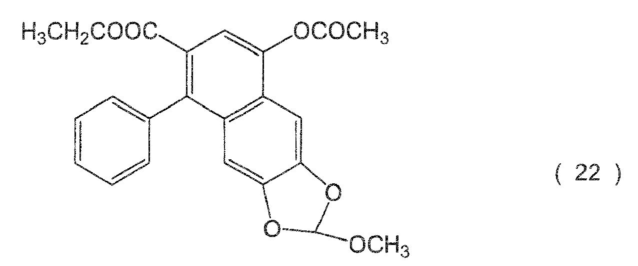

The compound of the above formula (22) was dispersed into 80 ml of methanol. 300 ml of an aqueous solution of 26.7 g (667.8 mmol) of sodium hydroxide was added to this solution and refluxed for 3 hours. After the reaction, toluene and tetrahydrofuran were added, the reaction product was washed with concentrated hydrochloric acid and then with water, the solvent was removed, and the obtained product was purified by reslurrying with toluene to obtain a compound represented by the following formula (23) as 11.3 g (33.4 mmol, yield rate of 90 %) of a yellow solid.

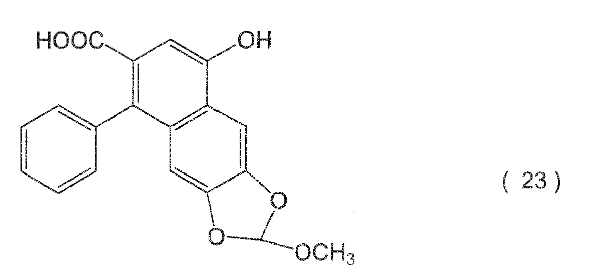

The compound of the above formula (23) and 9.3 g (73.5 mmol) of benzyl chloride were dissolved in 160 ml of N,N-dimethylformamide. 11.5 g (83.5 mmol) of potassium carbonate was added to this solution, heated to 60°C and stirred for 3 hours. After the reaction, toluene was added, the reaction product was washed in water, and the solvent was removed to obtain a compound represented by the following formula (24) as 17.1 g (33.1 mmol, yield rate of 99 %) of yellow oil.

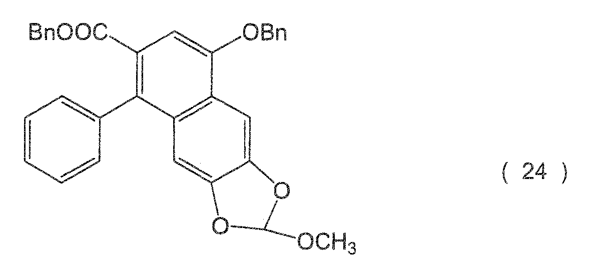

The compound of the above formula (24) was dispersed into 150 ml of isopropyl alcohol. 120 ml of an aqueous solution of 19.9 g (496.5 mmol) of sodium hydroxide was added to this solution and refluxed for 3 hours. After the reaction, toluene was added, the reaction product was washed with concentrated hydrochloric acid and then with water, the solvent was removed, and the obtained product was purified by reslurrying with toluene to obtain a compound represented by the following formula (25) as 12.7 g (29.6 mmol, yield rate of 89 %) of a yellow solid.

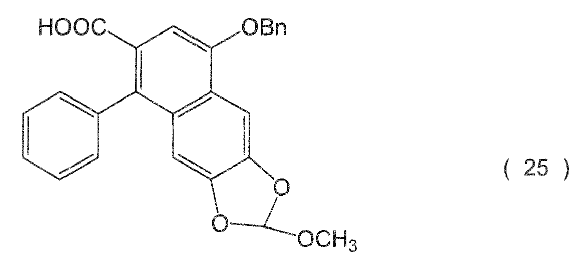

The compound of the above formula (25) was dispersed into 350 ml of toluene. 9.0 g (88.8 mmol) of triethylamine and 10.6 g (38.5 mmol) of diphenylphosphorylazide were added to this solution and stirred at room temperature for 2 hours. 6.8 g (148.0 mmol) of ethanol was added to this solution to carry out a reaction at 70°C for 2 hours. Thereafter, 100 ml of ethanol was added to this solution, and then 16.6 g (296.0 mmol) of potassium hydroxide was added and refluxed for 5 hours. After the reaction, ethanol was distilled off at normal pressure, tetrahydrofuran was added, the obtained product was washed in water, and the solvent was removed to obtain a compound represented by the following formula (26) as 11.8 g (29.6 mmol, yield rate of 100 %) of a yellow solid.

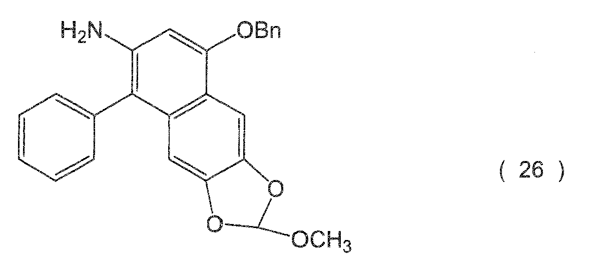

The compound of the above formula (26) was dispersed into 500 ml of acetonitrile, and 93.5 g (148.0 mmol) of a 6 % hydrochloric acid aqueous solution was added to the dispersion and cooled to 0 to 5°C. 18.4 g (88.8 mmol) of a 33 % sodium nitrite aqueous solution was added to this solution and stirred for 30 minutes. 51.5 g (148. mmol) of a 50 % potassium iodide aqueous solution was added to this solution and stirred at room temperature for 5 hours. After the reaction, toluene was added, the reaction product was washed in water, the solvent was removed, and the obtained product was purified by column chromatography to obtain a compound represented by the following formula (27) as 10.6 g (20.7 mmol, yield rate of 70 %) of a yellow solid.

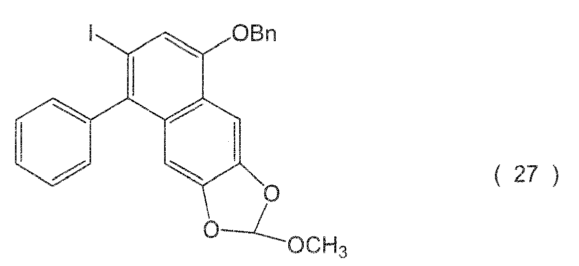

The compound of the above formula (27) was dispersed into 600 ml of toluene and cooled to -30°C. 15.6 ml (24.9 mmol) of n-butyl lithium (1.6M hexane solution) was added dropwise to this solution and stirred for 30 minutes. 8.0 ml of a toluene solution of 4.0 g (25.9 mmol) of 3,3,5,5-tetramethylcyclohexanone was added dropwise to this solution and stirred at 0°C for 3 hours. After the reaction, toluene was added, the reaction product was washed in water, the solvent was removed, and the obtained product was purified by reslurrying with methanol to obtain a compound, represented by the following formula (28) as 7.3 g (13.5 mmol, yield rate of 65 %) of a yellow solid.

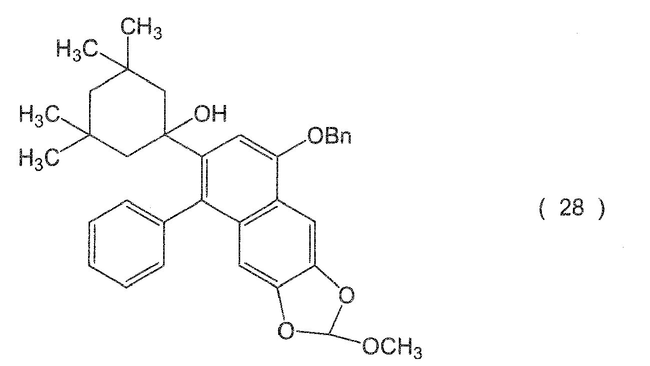

The compound of the above formula (28) was dissolved in 200 ml of tetrahydrofuran, and 3.4 g (54.0 mmol) of ammonium formate and 3.8 g of 5 % palladium carbon were added and stirred at room temperature for 8 hours. After the reaction, filtration was carried out, toluene was added, the reaction product was washed in water, the solvent was removed, and the obtained product was purified by reslurrying with toluene to obtain a compound represented by the following formula (29) as 5.5 g (12.2 mmol, yield rate of 90 %) of a yellow solid.

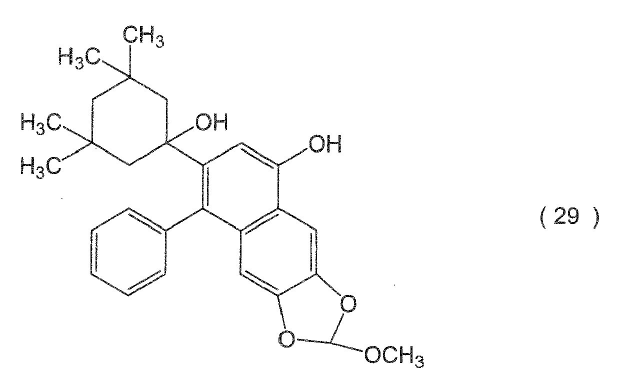

The compound of the above formula (29) was dissolved in 150 ml of toluene and heated to 90°C. 7.0 g (36.6 mmol) of p-toluensulfonic acid was added to this solution and refluxed for 3 hours. After the reaction, the reaction product was washed in water, and the solvent was removed to obtain a naphthol compound represented by the following formula (30) as 4.0 g (9.2 mmol, yield rate of 75 %) of a yellow solid.

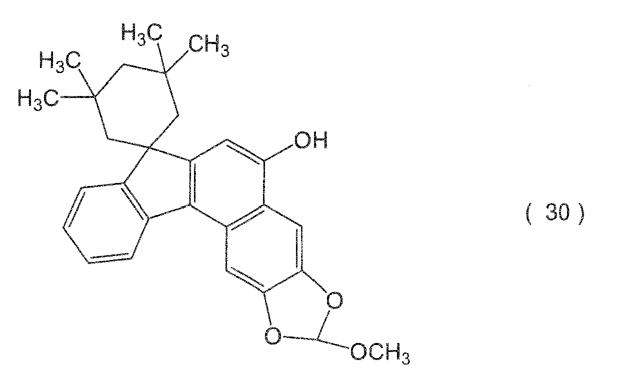

The elemental analysis values of this product were 78.15 % of C, 7.04 % of H and 0.00 % of N which were almost equal to the calculated values of C₂₈H₃₀O₄ (C: 78.11%, H: 7.02 %, N: 0.00 %).

When the proton nuclear magnetic resonance spectrum of this product was measured, it showed a 22H peak based on an alkyl group at δ of around 0.5 to 4.5 ppm and 8H peaks based on a hydroxyl group and an aromatic proton at δ of around 5.0 to 9.0 ppm

Further, when the ¹³C-nuclear magnetic resonance spectrum was measured, it showed a peak based on the Carbon of an aromatic ring at δ of around 110 to 160 ppm and a peak based on the carbon of an alkyl group at δ of around 20 to 80 ppm.

It was confirmed from these results that the isolated product was a naphthol compound represented by the above formula (30).

This compound is a naphthol compound used in the above Example 1.

### Examples 46 to 66

Naphthol compounds shown in Table 8 were synthesized in the same manner as in Example 45. When the structures of the obtained products were analyzed by using the same structure confirming means as in Example 45, it was confirmed that they were naphthol compounds used in Examples shown in Table 8. Table 8 shows the elemental analysis values, calculated values obtained from the structural formulas of the compounds and characteristic ¹H-NMR spectra of these compounds.

**Table 8**

| Example No. | Chromene Compound No.* | Elemental analysis values | | | | | | ¹H-NMR (NMR) |
|---|---|---|---|---|---|---|---|---|
| | | Experimental values | | | Calculated values | | | |
| | | C | H | N | C | H | N | |
| 45 | 1 | 78.15 | 7.04 | 0.00 | 78.11 | 7.02 | 0.00 | δ5.0-9.0 8H δ0.5-4.5 22H |
| 46 | 2 | 68.59 | 5.72 | 2.77 | 68.56 | 5.75 | 2.67 | δ5.0-9.0 7H δ0.5-4.5 23H |
| 47 | 3 | 75.99 | 7.21 | 0.00 | 75.92 | 7.22 | 0.00 | δ5.0-9.0 8H δ0.5-4.5 26H |
| 48 | 7 | 76.75 | 7.22 | 2.84 | 76.67 | 7.26 | 2.88 | δ5.0-9.0 8H δ0.5-4.5 27H |
| 49 | 8 | 78.55 | 7.60 | 3.22 | 78.52 | 7.50 | 3.16 | δ5.0-9.0 8H δ0.5-4.5 25H |
| 50 | 9 | 77.78 | 6.79 | 0.00 | 77.86 | 6.78 | 0.00 | δ5.0-9.0 8H δ0.5-4.5 20H |
| 51 | 10 | 80.44 | 6.65 | 0.00 | 80.46 | 6.55 | 0.00 | δ5.0-9.0 8H δ0.5-4.5 24H |
| 52 | 11 | 80.56 | 6.77 | 0.00 | 80.60 | 6.76 | 0.00 | δ5.0-9.0 13H δ0.5-4.5 21H |
| 53 | 12 | 78.43 | 7.22 | 0.00 | 78.35 | 7.26 | 0.00 | δ5.0-9.0 24H δ0.5-4.5 |
| 54 | 13 | 76.48 | 7.88 | 5.77 | 76.51 | 7 87 | 5.76 | δ5.0-9.0 8H δ0.5-4.5 30H |
| 55 | 14 | 78.43 | 7.21 | 0.00 | 78.35 | 7.26 | 0.00 | δ5.0-9.0 8H δ0.5-4.5 24H |
| 56 | 15 | 76.94 | 7.44 | 2.79 | 76.92 | 7.46 | 2.80 | δ5.0-9.0 29H δ0.5-4.5 |
| 57 | 16 | 78.76 | 7.77 | 3.03 | 78.74 | 7.71 | 3.06 | δ5.0-9.0 8H δ0.5-4.5 27H |
| 58 | 17 | 78.33 | 7.30 | 0.00 | 78.85 | 7.26 | 0.00 | δ5-0-9.0 8H δ0.5-4.5 24H |
| 59 | 18 | 76.92 | 7.43 | 2.80 | 76.92 | 7.46 | 2.80 | δ5.0-9.0 8H δ0.5-4.5 29H |
| 60 | 19 | 78.63 | 7.46 | 0.00 | 78.57 | 7.47 | 0.00 | δ5.0-9.0 8H δ0.5-4.5 26H |
| 61 | 20 | 80.72 | 6.93 | 0.00 | 80.74 | 6.97 | 0.00 | δ5.0-9.0 13H δ0.5-4.5 23H |
| 62 | 21 | 74.80 | 7.44 | 2.66 | 74.83 | 7.42 | 2.64 | δ5.0-9.0 8H δ0.5-4.5 31H |
| 63 | 22 | 78.54 | 7.54 | 0.00 | 78.57 | 7.47 | 0.00 | δ5.0-9.0 8H δ0.5-4.5 26H |
| 64 | 23 | 78.56 | 7.50 | 0.00 | 78.57 | 7.47 | 0.00 | δ5.0-9.0 8H δ0.5-4.5 26H |
| 65 | 24 | 75.45 | 5.44 | 0.00 | 75.43 | 5.43 | 0.00 | δ5.0-9.0 8H δ0.5-4.5 10H |
| 66 | 25 | 77.77 | 5.69 | 0.00 | 77.70 | 5.74 | 0.00 | δ5.0-9.0 14H δ0.5-4.5 14H |

| | | | | | | | | |
|---|---|---|---|---|---|---|---|---|
| *Examples of chromene compounds produced by using the naphthol compounds of Examples | | | | | | | | |

### Effect of the Invention

Since the Chromene compound of the present invention develops a color of a neutral tint by itself, it can be used alone and hardly undergoes a color change at the time of fading and a color change at the time of deterioration. Further, since the chromene compound has little initial coloration, high color optical density, high double peak characteristic and high fading speed, an extremely excellent photochromic lens can be obtained from the chromene compound. Therefore, color can be controlled by mixing it with another photochromic compound, and even when it is mixed with another photochromic compound, it can exhibit excellent photochromic properties.

## Claims

1. A chromene compound having a skeleton represented by the following formula (1): 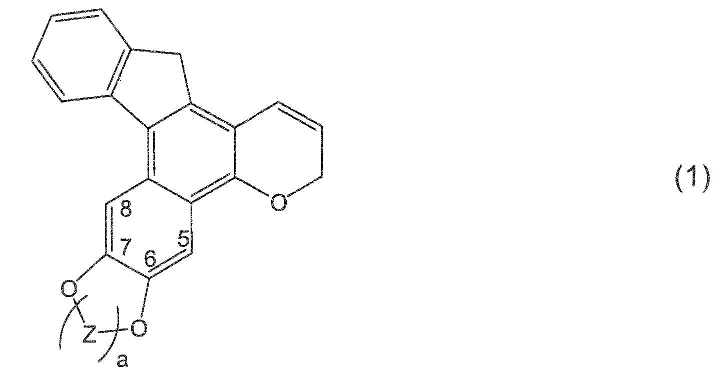 wherein Z is a group represented by any one of the following formulas: 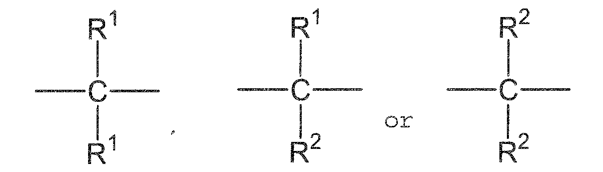 ,wherein R¹ is an electron donor group having a Hammett constant σₚ of less than -0.20, with the proviso that when there are a plurality of R¹' s, R¹'s may be the same or different, and R² is a group having a Hammett constant σₚ of -0.20 to 0, with the proviso that when there are a plurality of R²'s, R²'s may be the same or different.
and "a" is an integer of 1 to 3, with the proviso that when "a" is 2 or 3, Z's may be the same or different but 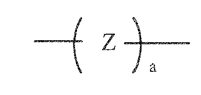 cannot be

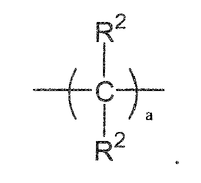

2. The chromene compound according to claim 1 which is represented by the following formula (2): 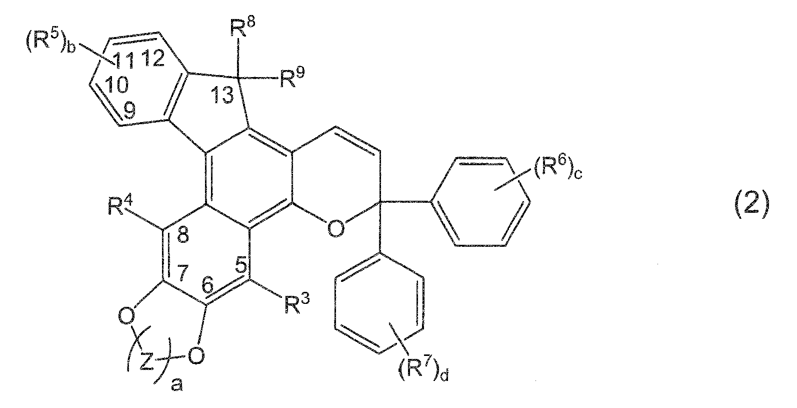 wherein Z and "a" are as defined in the above formula (1), R³ and R⁴ are each independently a hydrogen atom, hydroxyl group, alkyl group, haloalkyl group, alkenyl group, alkynyl group, cycloalkyl group, alkoxy group, aralkyl group, aralkoxy group, aryloxy group, aryl group, amino group, heterocyclic group having a nitrogen atom as a ring member hetero atom and bonded to the 5-position or 8-position carbon atom via the nitrogen atom, cyano group, nitro group, formyl group, hydroxycarbonyl group, alkylcarbonyl group, alkoxycarbonyl group or halogen atom, R⁵, R⁶ and R⁷ are each independently a hydroxyl group, alkyl group, haloalkyl group, alkenyl group, alkynyl group, cycloalkyl group, alkoxy group, aralkyl group, aralkoxy group, aryloxy group, aryl group, amino group, heterocyclic group having a nitrogen atom as a ring member hetero atom and bonded to a benzene ring bonded thereto via, the nitrogen atom, cyano group, nitro group, formyl group, hydroxycarbonyl group, alkylcarbonyl group, alkoxycarbonyl group or halogen atom, R⁸ and R⁹ are each independently a hydrogen atom, hydroxyl group, alkyl group, haloalkyl group, alkenyl group, alkynyl group, cycloalkyl group, alkoxy group, aralkyl group, aralkoxy group, aryloxy group, aryl group, amino group, heterocyclic group having a nitrogen atom as a ring member hetero atom and bonded to the 13-position carbon atom via the nitrogen atom, cyano group, nitro group, formyl group, hydroxycarbonyl group, alkylcarbonyl group, alkoxycarbonyl group or halogen atom, R⁸ and R⁹ may be bonded together to form a carbonyl group or aliphatic hydrocarbon ring together with the 13-position carbon atom, b is an integer of 0 to 4, and c and d are each independently an integer of 0 to 5, with the proviso that when b is 2 to 4, R⁵'s may be the same or different, and when c and d are each 2 to 5, R⁶'s and R⁷'s may be the same or different.

3. The chromene compound according to claim 2, wherein, in the chromene compound represented by the above formula (2), R⁸ and R⁹ are bonded together to form an aliphatic hydrocarbon ring together with the 13-position carbon atom, and the aliphatic hydrocarbon ring has 4 to 20 ring member carbon atoms and may have at least one substituent selected from the group consisting of alkyl group, haloalkyl group, cycloalkyl group, alkoxy group, amino group, aralkyl group, aryl group and halogen atom.

4. A photochromic curable composition comprising the chromene compound of any one of claims 1 to 3 and a polymerizable monomer.

5. A photochromic optical article having a polymer molded product containing the chromene compound of any one of claims 1 to 3 dispersed therein as a constituent member of the photochromic optical article.

6. An optical article comprising an optical substrate which is at least partially coated with a polymer film as a constituent part of the optical article, wherein the polymer film contains the chromene compound of any one of claims 1 to 3 dispersed therein.

7. A naphthol compound represented by the following formula (3): 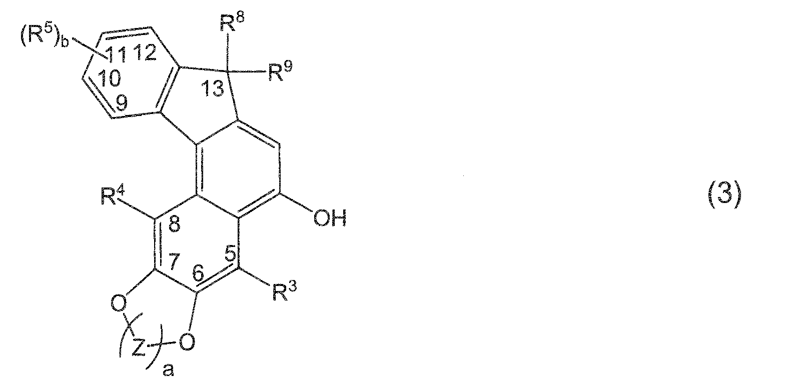 wherein Z and "a" are as defined in the above formula (1), and R³, R⁴, R⁵, R⁸ R⁹ and b are as defined in the above formula (2) .
